(19) **Europäisches Patentamt**
**European Patent Office**
**Office européen des brevets**

(11) **EP 4 773 156 A1**

(12) **EUROPEAN PATENT APPLICATION**
published in accordance with Art. 153(4) EPC

(43) Date of publication:
08.07.2026 Bulletin 2026/28

(21) Application number: 24862845.5

(22) Date of filing: 05.09.2024

(51) International Patent Classification (IPC):
$H01F\ 1/36^{(2006.01)}$     $B82Y\ 5/00^{(2011.01)}$
$B82Y\ 40/00^{(2011.01)}$     $C01G\ 49/08^{(2006.01)}$
$G01N\ 33/531^{(2006.01)}$     $G01N\ 33/543^{(2006.01)}$
$G01N\ 33/545^{(2006.01)}$     $H01F\ 1/00^{(2006.01)}$
$H01F\ 1/11^{(2006.01)}$     $H01F\ 1/34^{(2006.01)}$
$H01F\ 1/44^{(2006.01)}$

(52) Cooperative Patent Classification (CPC):
B82Y 5/00; B82Y 40/00; C01G 49/08;
G01N 33/531; G01N 33/543; G01N 33/545;
H01F 1/00; H01F 1/11; H01F 1/34; H01F 1/36;
H01F 1/44

(86) International application number:
PCT/JP2024/031799

(87) International publication number:
WO 2025/053193 (13.03.2025 Gazette 2025/11)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 07.09.2023 JP 2023145189
07.09.2023 JP 2023145346
03.09.2024 JP 2024151501

(71) Applicant: Canon Kabushiki Kaisha
Tokyo 146-8501 (JP)

(72) Inventors:
• YAMABI, Satoshi
Tokyo 146-8501 (JP)
• KAKEGAWA, Norishige
Tokyo 146-8501 (JP)
• SASAGURI, Daisuke
Tokyo 146-8501 (JP)
• ABE, Shigemoto
Tokyo 146-8501 (JP)

(74) Representative: WESER & Kollegen
Patentanwälte PartmbB
Radeckestraße 43
81245 München (DE)

(54) **MAGNETIC PARTICLE, MAGNETIC PARTICLE FOR ASSAY, AND METHOD FOR PRODUCING MAGNETIC PARTICLE**

(57) The purpose of the present application is to provide a magnetic particle which includes a secondary particle in which a plurality of primary particles, which each of the primary particles with a particle size which is conceived to exhibit superparamagnetism are aggregated, and the secondary particle exhibits a larger particle size.

[Means for Solving the Problem] The purpose is achieved by the magnetic particle which includes a secondary particle in which a plurality of primary particles are aggregated, wherein an average particle size of the secondary particle is 500 nm or more and 5,000 nm or less, and wherein an average particle size of the plurality of primary particles is 2 nm or more and 20 nm or less.

FIG. 1

**Description**

[Technical Field]

The present invention relates to a magnetic particle, a magnetic particle for assay, and a method of producing a magnetic particle.

[Background Art]

A magnetic particle containing $Fe_3O_4$ has been applied in biomedical fields, such as immunoassay, separation and purification of biomolecules, and drug delivery.

$Fe_3O_4$ particles applied in such biomedical fields need to have high handleability by an external magnetic field. Primary magnetic properties of $Fe_3O_4$ particles closely related to the handleability by an external magnetic field are remanent magnetization and saturation magnetization (hereinafter simply referred to as "magnetization"), and each magnetization depends on particle size of each of the $Fe_3O_4$ particles.

In order to easily control aggregation and dispersion of $Fe_3O_4$ particles in a medium by an external magnetic field, it is required to reduce the remanent magnetization of the $Fe_3O_4$ particles, which is the magnetization when the application of the external magnetic field is stopped. It has been known that the remanent magnetization of the $Fe_3O_4$ particles decreases as the particle size decreases, and when the particle size becomes 20 nm or less, the remanent magnetization becomes zero, that is, superparamagnetism is exhibited.

In Non Patent Literature 1, there is a disclosure of a $Fe_3O_4$ cluster synthesized by adding sodium acrylate to an ethylene glycol solution of iron(III) chloride hexahydrate for the purpose of reducing the particle size of each of primary particles. In Non Patent Literature 2, there is a disclosure of a $Fe_3O_4$ cluster synthesized by adding trisodium citrate to an ethylene glycol solution of iron(III) chloride anhydrous for the purpose of reducing a particle size of each of primary particles. Both of those $Fe_3O_4$ clusters have a primary particle size of 20 nm or less, and hence the $Fe_3O_4$ clusters are superparamagnetic substances.

[Citation List]

[Non Patent Literature]

NPL 1: S. Xuan, Y. X. J. Wang, J. C. Yu, K. C. F. Leung, Chemistry of Materials, vol. 21, p. 5079-5087 (2009).
NPL 2: J. Liu, Z. Sun, Y. Deng, Y. Zou, C. Li, X. Guo, L. Xiong, Y. Gao, F. Li, D. Zhao, Angewandte Chemie International Edition, vol. 48, p. 5875-5879 (2009).

[Summary of Invention]

[Technical Problem]

The present invention is directed to providing a magnetic particle including a secondary particle in which a plurality of primary particles each having a particle size conceived to exhibit superparamagnetism are aggregated, the secondary particle having a larger particle size.

[Solution to Problem]

In order to solve the above-mentioned problem, according to the present invention, there is provided a magnetic particle including a secondary particle in which a plurality of primary particles are aggregated, wherein an average particle size of the secondary particle is 500 nm or more and 5,000 nm or less, and wherein an average particle size of the primary particles is 2 nm or more and 20 nm or less.

In addition, according to the present invention, there is provided a magnetic particle for assay, the magnetic particle including: the above-mentioned magnetic particle; and a resin layer arranged outside the magnetic particle.

In addition, according to the present invention, there is provided a method of producing a magnetic particle including a secondary particle in which a plurality of primary particles are aggregated, the method including: a step of obtaining an iron(III)-containing solution; a step of aging the iron(III)-containing solution; a step of obtaining a mixture in which the iron(III)-containing solution obtained in the step of aging and a precipitant are mixed; and a step of heating the mixture.

[Advantageous Effects of Invention]

According to the present invention, the $Fe_3O_4$ cluster that achieves both of superparamagnetism and large magnetization can be provided.

[Brief Description of Drawings]

[Fig. 1]

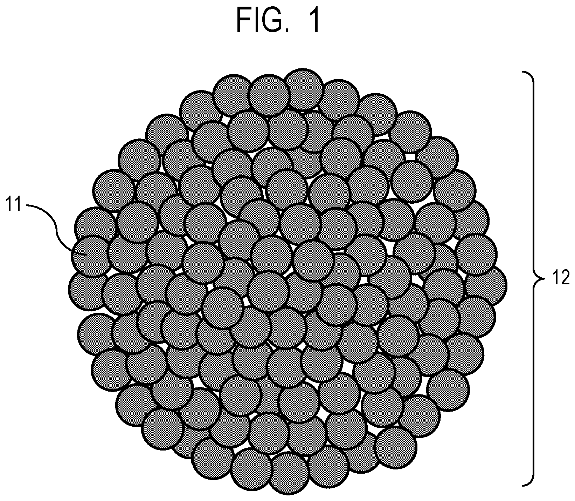

Fig. 1 is a schematic view of a structure of a magnetic particle ($Fe_3O_4$ cluster) according to an embodiment of the present invention.
[Fig. 2]

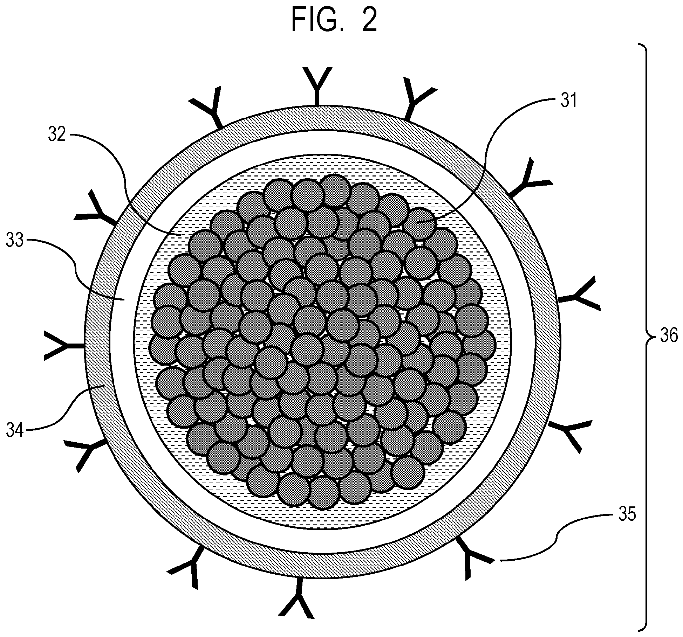

Fig. 2 is a schematic view of a magnetic particle for assay according to an embodiment of the present invention.
[Fig. 3]

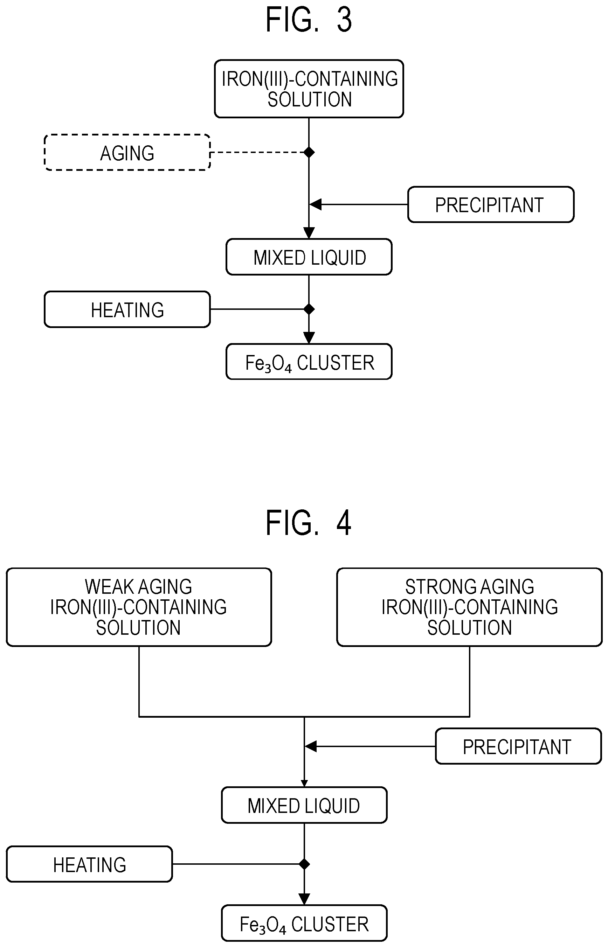

Fig. 3 is a synthesis flow of a $Fe_3O_4$ cluster.
[Fig. 4]
Fig. 4 is a synthesis flow of a $Fe_3O_4$ cluster by a blend method.
[Fig. 5A]

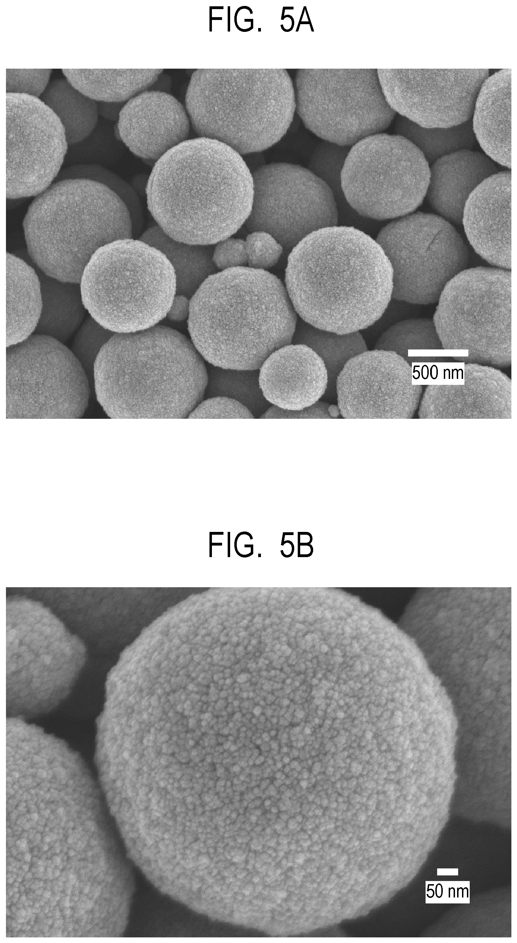

Fig. 5A is an SEM image (low magnification) of a $Fe_3O_4$ cluster produced in Example 1.
[Fig. 5B]
Fig. 5B is an SEM image (high magnification) of the $Fe_3O_4$ cluster produced in Example 1.
[Fig. 6A]

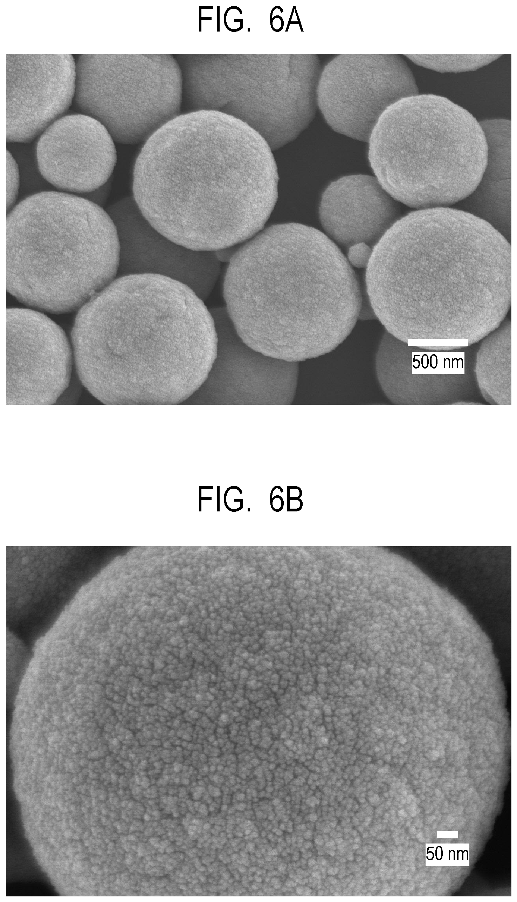

Fig. 6A is an SEM image (low magnification) of a $Fe_3O_4$ cluster produced in Example 2.
[Fig. 6B]
Fig. 6B is an SEM image (high magnification) of the $Fe_3O_4$ cluster produced in Example 2.
[Fig. 7A]

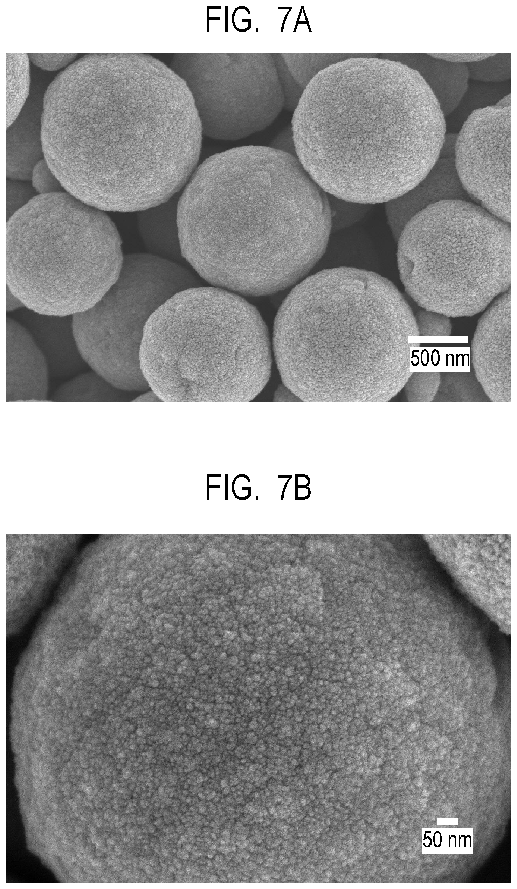

Fig. 7A is an SEM image (low magnification) of a $Fe_3O_4$ cluster produced in Example 6.
[Fig. 7B]
Fig. 7B is an SEM image (high magnification) of the $Fe_3O_4$ cluster produced in Example 6.
[Fig. 8A]

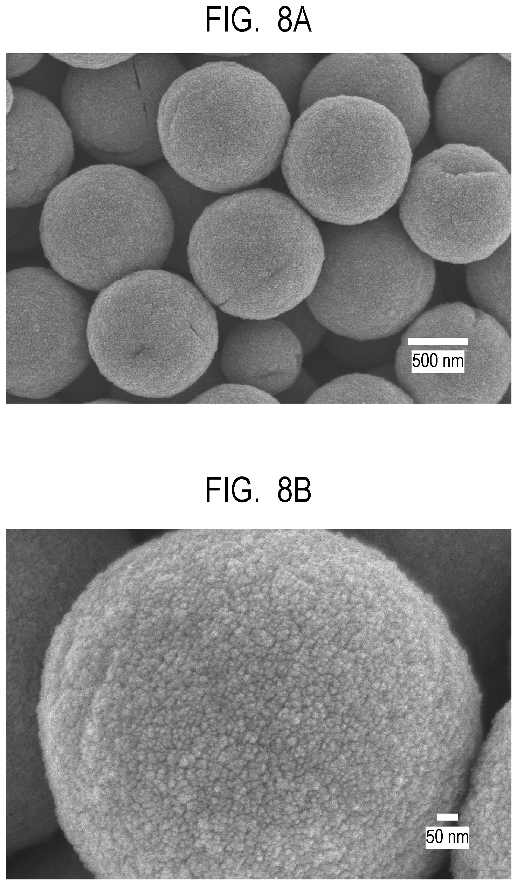

Fig. 8A is an SEM image (low magnification) of a $Fe_3O_4$ cluster produced in Example 8.
[Fig. 8B]
Fig. 8B is an SEM image (high magnification) of the $Fe_3O_4$ cluster produced in Example 8.
[Fig. 9A]

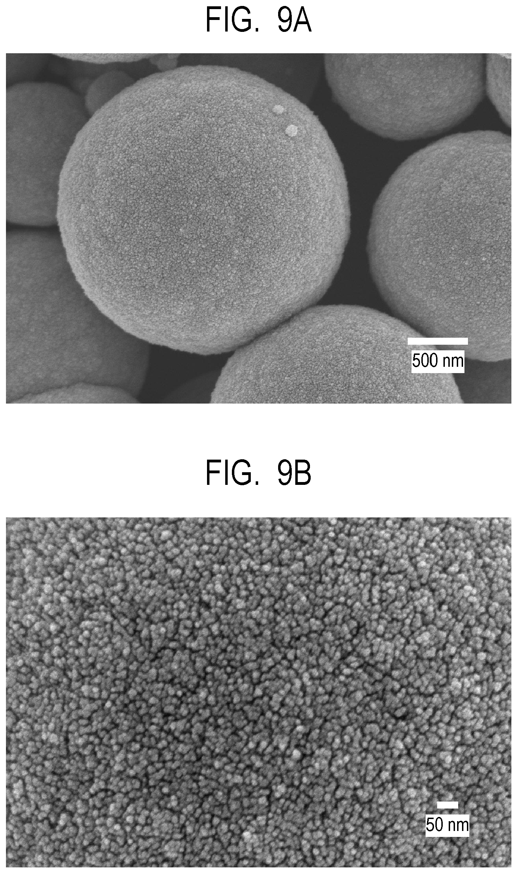

Fig. 9A is an SEM image (low magnification) of a $Fe_3O_4$ cluster produced in Example 14.
[Fig. 9B]
Fig. 9B is an SEM image (high magnification) of the $Fe_3O_4$ cluster produced in Example 14.
[Fig. 10A]

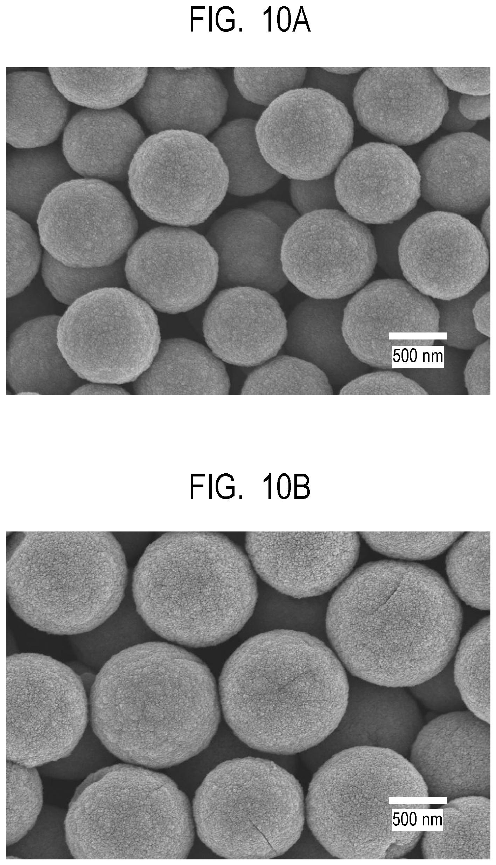

Fig. 10A is an SEM image of a $Fe_3O_4$ cluster produced in Example 22.
[Fig. 10B]
Fig. 10B is an SEM image of a $Fe_3O_4$ cluster produced in Example 23.
[Fig. 10C]

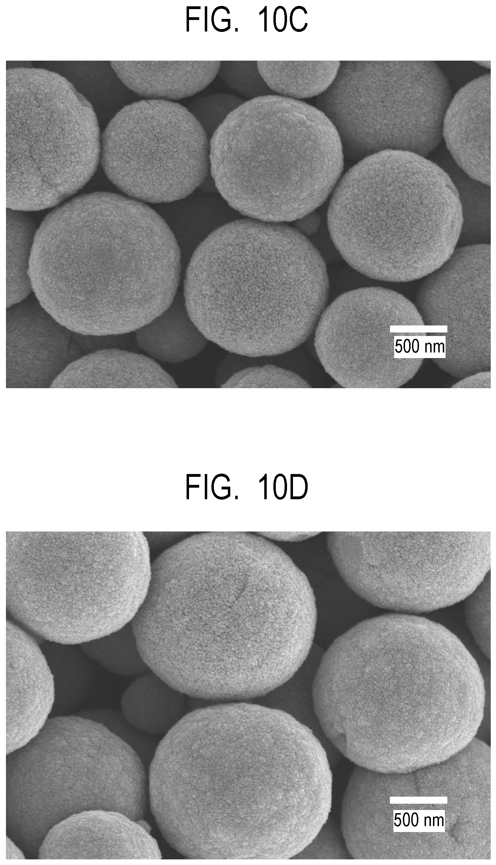

Fig. 10C is an SEM image of a $Fe_3O_4$ cluster produced in Example 24.
[Fig. 10D]
Fig. 10D is an SEM image of a $Fe_3O_4$ cluster produced in Example 25.
[Fig. 10E]

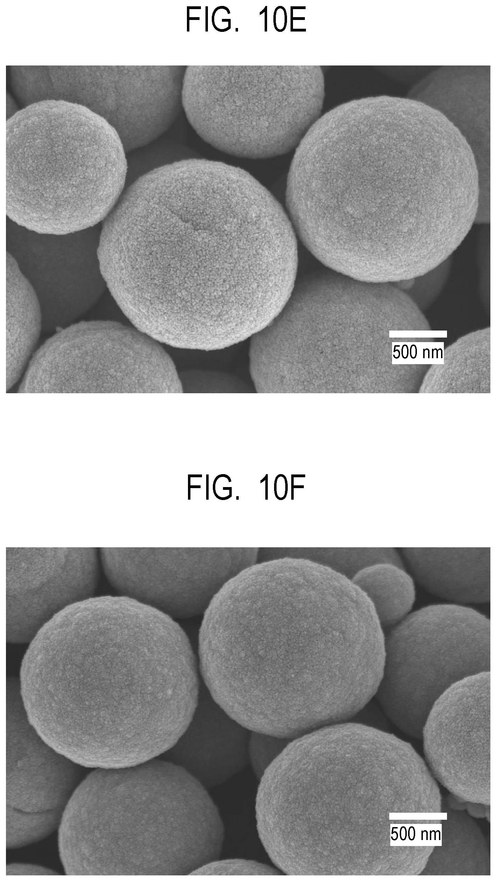

Fig. 10E is an SEM image of a $Fe_3O_4$ cluster produced in Example 26.
[Fig. 10F]
Fig. 10F is an SEM image of a $Fe_3O_4$ cluster produced in Example 27.
[Fig. 10G]

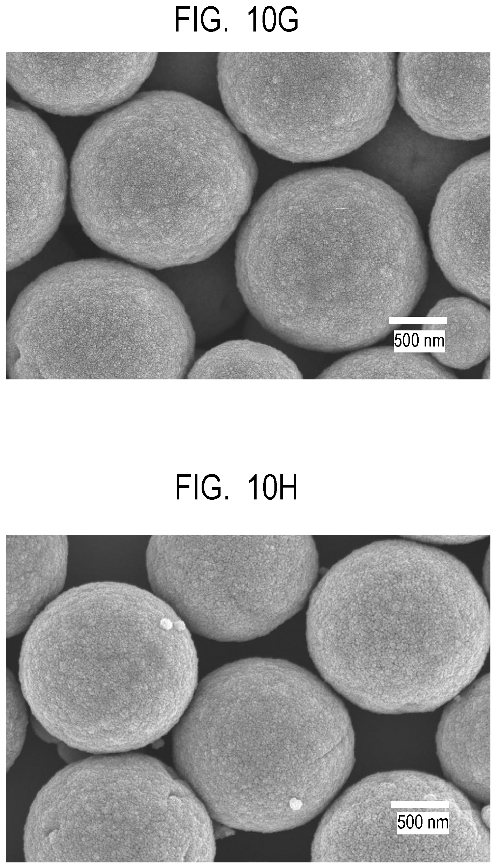

Fig. 10G is an SEM image of a $Fe_3O_4$ cluster produced in Example 28.
[Fig. 10H]
Fig. 10H is an SEM image of a $Fe_3O_4$ cluster produced in Example 29.
[Fig. 11A]

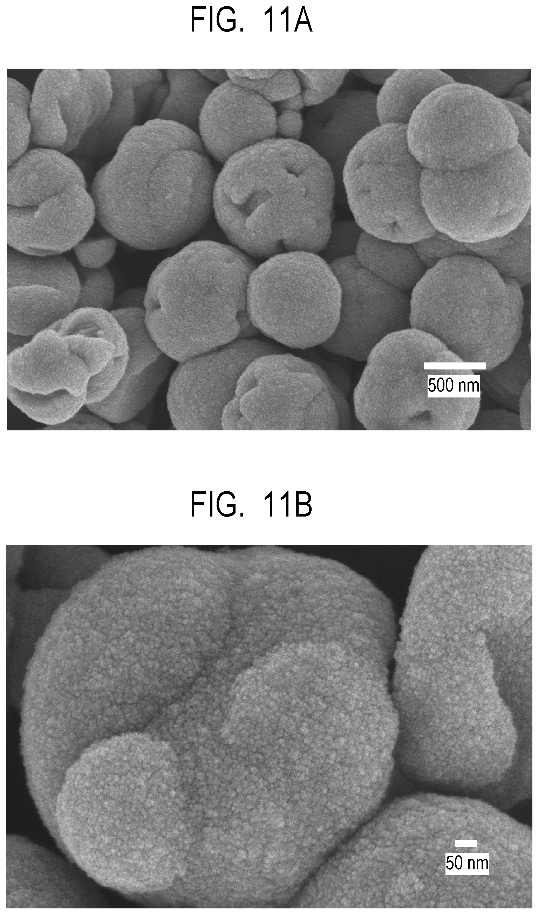

Fig. 11A is an SEM image (low magnification) of a $Fe_3O_4$ cluster produced in Comparative Example 2.
[Fig. 11B]
Fig. 11B is an SEM image (high magnification) of the $Fe_3O_4$ cluster produced in Comparative Example 2.

[Fig. 12A]

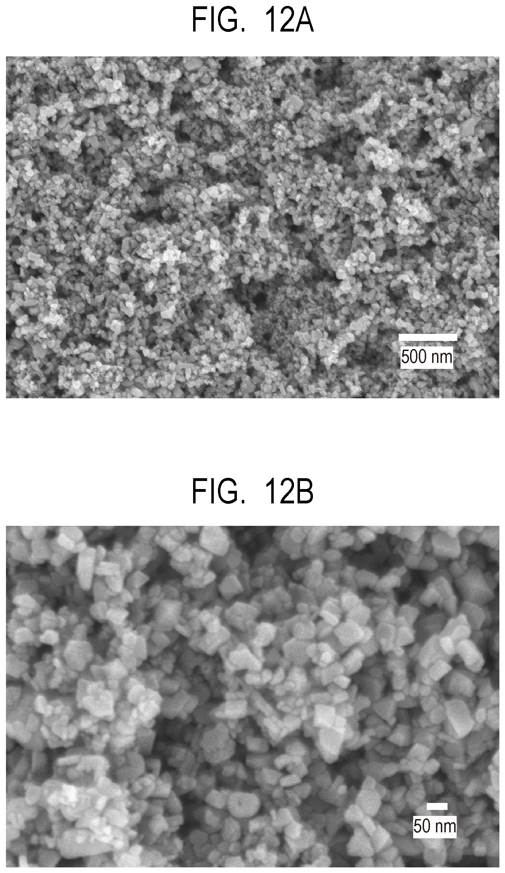

Fig. 12A is an SEM image (low magnification) of a $Fe_3O_4$ cluster produced in Comparative Example 3.
[Fig. 12B]
Fig. 12B is an SEM image (high magnification) of the $Fe_3O_4$ cluster produced in Comparative Example 3.
[Fig. 13A]

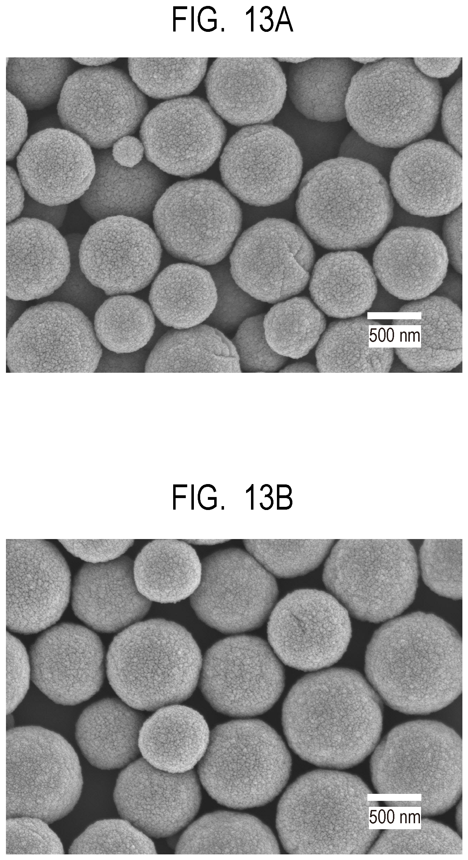

Fig. 13A is an SEM image of a $Fe_3O_4$ cluster produced in Synthesis Example 1.
[Fig. 13B]
Fig. 13B is an SEM image of a $Fe_3O_4$ cluster produced in Example 30.
[Fig. 13C]

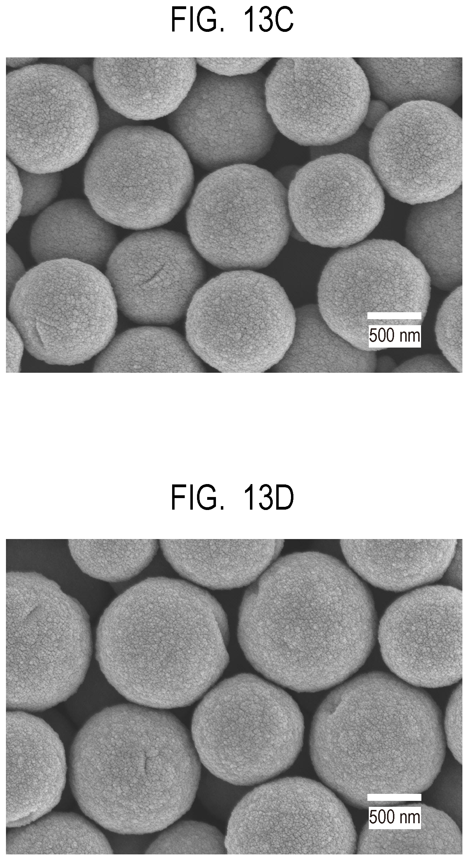

Fig. 13C is an SEM image of a $Fe_3O_4$ cluster produced in Example 31.
[Fig. 13D]
Fig. 13D is an SEM image of a $Fe_3O_4$ cluster produced in Example 32.
[Fig. 13E]

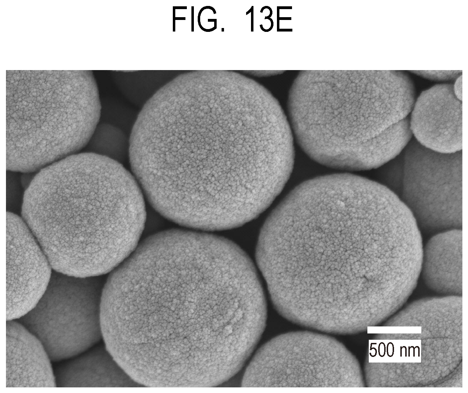

Fig. 13E is an SEM image of a $Fe_3O_4$ cluster produced in Synthesis Example 2.
[Fig. 14]

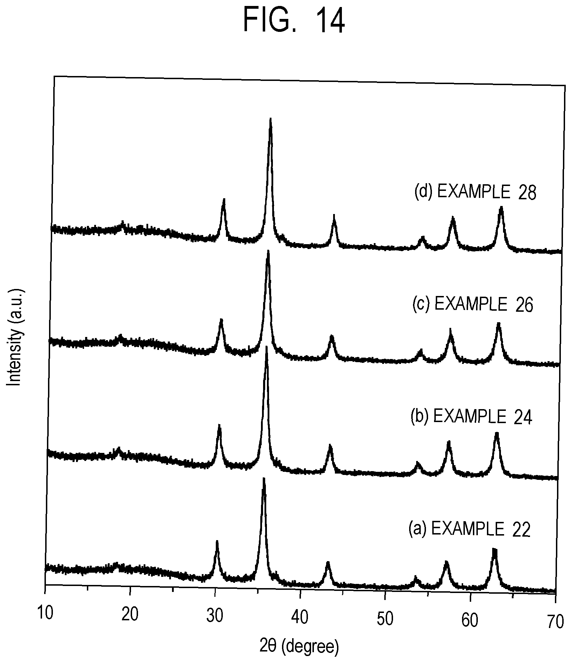

Fig. 14 is XRD patterns of produced particles: (a) Example 22, (b) Example 24, (c) Example 26, and (d) Example 28.
[Fig. 15A]

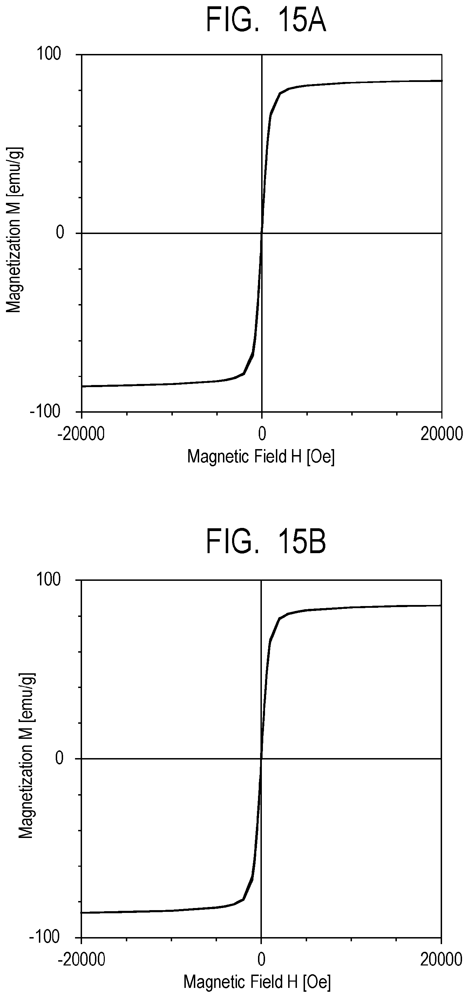

Fig. 15A is a hysteresis curve of the particles produced in Example 22.
[Fig. 15B]
Fig. 15B is a hysteresis curve of the particles produced in Example 24.
[Fig. 15C]

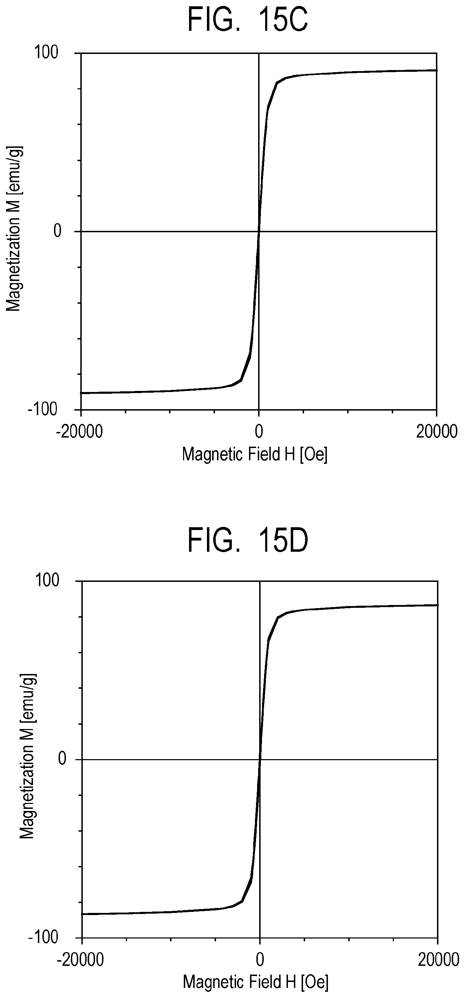

Fig. 15C is a hysteresis curve of the particles produced in Example 26.
[Fig. 15D]
Fig. 15D is a hysteresis curve of the particles produced in Example 28.

[Description of Embodiments]

Embodiments of the present invention are described in detail. In the present invention, the terms "$Fe_3O_4$ cluster" and "secondary particle" are synonymous.

[First Embodiment]

The first embodiment of the present invention relates to a magnetic particle.

First, a problem in this embodiment is described. In order to enable rapid movement and transportation of $Fe_3O_4$ particles in a medium by an external magnetic field in various tests each using a magnetic particle such as an optical waveguide-type test system to be described later, the $Fe_3O_4$ particles need to have large magnetization. In general, magnetization per particle of a magnetic particle increases as its particle size increases. Accordingly, when the particle size of the $Fe_3O_4$ particle is increased, the magnetization per particle of the $Fe_3O_4$ particle can be increased.

Thus, it is difficult to obtain $Fe_3O_4$ particles that achieve both of superparamagnetism and large magnetization because a reduction in particle size for superparamagnetism, which is a requirement for $Fe_3O_4$ particles having high handleability by an external magnetic field, and an increase in particle size for large magnetization are in a trade-off relationship.

A $Fe_3O_4$ cluster that is a secondary particle in which a plurality of $Fe_3O_4$ primary particles of several tens of nanometers are aggregated has been known. When, while particle sizes of the primary particles of the $Fe_3O_4$ cluster are kept small, a number of such primary particles to be aggregated is controlled, that is, the particle size of the secondary particle is controlled, a $Fe_3O_4$ cluster that achieves both of superparamagnetism and large magnetization can be obtained.

Meanwhile, the $Fe_3O_4$ clusters produced by the methods of Non Patent Literatures 1 and 2 described above both have primary particle sizes of 20 nm or less, and hence the $Fe_3O_4$ clusters are superparamagnetic substances. However, the particle size of the secondary particle is 280 nm at the maximum in Non Patent Literature 1 and 410 nm at the maximum in Non Patent Literature 2, and hence the magnetization per particle of each of those $Fe_3O_4$ clusters is small. Accordingly, those $Fe_3O_4$ clusters are not suitable for applications each requiring rapid movement and transportation of a magnetic particle in a medium by a magnetic field. The $Fe_3O_4$ cluster is required to have a larger particle size for rapid movement and transportation in a medium by an external magnetic field.

A magnetic particle according to an embodiment of the present invention is a magnetic particle including a secondary particle in which a plurality of primary particles are aggregated, wherein an average particle size of the secondary particle is 500 nm or more and 5,000 nm or less, and wherein an average particle size of the primary particles is 2 nm or more and 20 nm or less.

According to the magnetic particle according to this embodiment, the $Fe_3O_4$ cluster that can achieve both superparamagnetism and large magnetization can be provided. When the magnetic particle is used for an assay, particularly an immunoassay, the magnetic particle for assay in a solution can be collected in a shorter time period than the related-art products, and hence work efficiency can be significantly improved. In addition, the magnetic particle for assay can be collected even in a high-viscosity solution, and hence the immunoassay of the high-viscosity solution can be performed.

Details are described below.

The magnetic particle according to this embodiment is a magnetic particle including a secondary particle in which a plurality of primary particles are aggregated.

A structure of the magnetic particle ($Fe_3O_4$ cluster) according to this embodiment is illustrated in Fig. 1. In the magnetic particle according to this embodiment, the secondary particle preferably contains $Fe_3O_4$. The $Fe_3O_4$ cluster according to this embodiment is a secondary particle 12 in which a plurality of $Fe_3O_4$ primary particles 11 are aggregated. In the magnetic particle according to this embodiment, the shape of the secondary particle is preferably spherical. The shape of the $Fe_3O_4$ cluster is preferably spherical, though the shape depends on applications. The term "sphere" in this embodiment (Fig. 5A, Fig. 5B, Fig. 6A, Fig. 6B, Fig. 7A, Fig. 7B, Fig. 8A, Fig. 9A, Fig. 10A to Fig. 10H, Fig. 13A to Fig. 13E, and Fig. 15A and Fig. 15B) refers to a substantial sphere, such as a "round shape" or a "ball-like shape," and is not limited to a true sphere. The term also encompasses a sphere having anisotropy or a sphere partially having a dent or a crack. The shape of the $Fe_3O_4$ cluster according to this embodiment may be recognized by observing the secondary particle with a scanning electron microscope (SEM).

As described above, the magnetic properties of the $Fe_3O_4$ cluster depend on the particle size of each of the primary particles of the $Fe_3O_4$ cluster (primary particle size) and the particle size of the secondary particle thereof (secondary particle size). The remanent magnetization of the $Fe_3O_4$ cluster decreases as the primary particle size decreases, and when the primary particle size becomes 20 nm or less, the remanent magnetization becomes zero, that is, so-called superparamagnetism is exhibited. In addition, the number of the primary particles to be aggregated, that is, the secondary particle size has a correlation with the magnetization of the $Fe_3O_4$ cluster, and the magnetization of the $Fe_3O_4$ cluster increases as the secondary particle size increases. That is, a magnetic particle having a larger secondary size diameter has larger magnetization (e.g., saturation magnetization). A $Fe_3O_4$ cluster in which the primary particles are aggregated so that the size diameter becomes 500 nm or more has large magnetization.

In the magnetic particle according to this embodiment, the magnetism of the secondary particle is superparamagnetism preferably. The magnetic properties and magnetization of the secondary particle of the $Fe_3O_4$ cluster in the present invention may each be measured with, for example, a commercially available vibrating sample magnetometer (VSM) or superconducting quantum interference device (SQUID).

The primary particle size and secondary particle size of the $Fe_3O_4$ cluster in this embodiment are number-average particle sizes measured from scanning electron microscope (SEM) images. For each of Examples and Comparative Examples, an average value of particle sizes of 50 primary particles and an average value of particle sizes of 50 secondary particles, the particles being randomly sampled from the SEM image of the produced $Fe_3O_4$ cluster, were calculated. In addition, with regard to the primary particle size, the calculation was performed by using Scherrer equation from half-width of diffraction peak of X-ray diffraction (XRD). With regard to the secondary particle size, median diameters D50 and D90 were calculated from a number-based particle size distribution measured by dynamic light scattering (DLS).

In the magnetic particle according to this embodiment, an average of the secondary particle sizes is 500 nm or more and 5,000 nm or less, and an average of the secondary particle sizes is preferably 500 nm or more and 3,000 nm or less. In the magnetic particle according to this embodiment, the secondary particle size (upper limit value) is 5,000 nm or less, preferably 4,000 nm or less, more preferably 3,000 nm or less, still more preferably 2,400 nm or less. In addition, in the magnetic particle according to this embodiment, the secondary particle size (lower limit value) is preferably 500 nm or more, more preferably 700 nm or more, still more preferably 800 nm or more, particularly preferably 1,000 nm or more. With this configuration, a magnetic particle having large magnetization (e.g., large saturation magnetization) can be obtained. For example, a magnetic particle having a particle size (e.g., a secondary particle size of 800 nm) twice as large as that of the related-art product (e.g., a secondary particle size of 400 nm) can have a saturation magnetization about 8 times as large as that of the related-art product, and a magnetic particle having a particle size (e.g., a secondary particle size of 2,400 nm) 6 times as large as that of the related-art product can have a saturation magnetization more than 200 times as large as that of the related-art product.

In the magnetic particle according to this embodiment, the average of the primary particle sizes (average particle size) is 2 nm or more and 20 nm or less. In the magnetic particle according to this embodiment, the primary particle size (upper limit value) is 20 nm or less, preferably 19 nm or less, more preferably 18 nm or less, still more preferably 16 nm or less. In addition, in the magnetic particle according to this embodiment, the primary particle size (lower limit value) is 2 nm or more, preferably 4 nm or more, more preferably 7 nm or more, still more preferably 10 nm or more, particularly preferably 12 nm or more. With this configuration, the magnetic particle according to this embodiment has superparamagnetism, and the remanent magnetization can be 0 (zero).

In the magnetic particle according to this embodiment, the secondary particle typically contains $Fe_3O_4$, but may

contain any other iron oxide, such as $Fe_2O_3$ or $FeO$, to the extent that the effects in this embodiment can be obtained. In addition, the magnetic particle is not limited to a simple substance and may be a particle of a mixture containing a substance having magnetism such as ferrite. In the magnetic particle according to this embodiment, the secondary particle is $Fe_3O_4$ preferably. The magnetic particle according to this embodiment is preferably formed only of the $Fe_3O_4$ cluster, and is basically preferably free of any other composition or structure such as $\gamma$-$Fe_2O_3$. The fact that the magnetic particle is formed only of $Fe_3O_4$ and is free of any other substance such as $\gamma$-$Fe_2O_3$ may be recognized by X-ray diffraction (XRD).

[Application Example]

An application example in this embodiment relates to a magnetic particle for assay. The magnetic particle for assay according to the embodiment of the present invention preferably includes the magnetic particle according to this embodiment and a resin layer arranged outside the magnetic particle.

The magnetic particle for assay according to this embodiment is described with reference to Fig. 2.

A magnetic particle 36 for assay according to this embodiment preferably includes a silica layer between a magnetic particle and a resin layer. A $Fe_3O_4$ cluster 31 serving as a core is coated with an inorganic layer 32.

In order to secure smoothness of a surface of the $Fe_3O_4$ cluster 31, the $Fe_3O_4$ cluster is coated with the inorganic layer 32. A silica layer is preferred as the inorganic layer 32. Silica is chemically stable, and for example, a uniform and smooth layer can be formed on the surface of the $Fe_3O_4$ cluster 31 by a wet method such as Stober method. The outside of the inorganic layer 32 is coated with a resin layer.

In the magnetic particle for assay according to this embodiment, the resin layer preferably include at least a first resin layer 33 and a second resin layer 34. In the magnetic particle 36 for assay according to this embodiment, the first resin layer 33 and the second resin layer 34 are formed outside the inorganic layer 32. The first resin layer 33 is formed in order to protect the $Fe_3O_4$ cluster 31. For example, the silica layer formed by the Stober method has a porous structure, and is hence insufficient for chemical protection of $Fe_3O_4$. In the magnetic particle for assay according to this embodiment, the first resin layer 33 is preferably a hydrophobic resin layer. The first resin layer 33 is preferably a styrene-based resin having high chemical stability and high hydrophobicity.

In the magnetic particle for assay according to this embodiment, the second resin layer 34 is arranged outside the first resin layer 33 preferably, and the second resin layer 34 is a polyglycidyl methacrylate layer preferably. The magnetic particle 36 for assay according to this embodiment further includes a ligand bound to the surface of the resin layer preferably, and the ligand is preferably an antibody 35. The second resin layer 34 is arranged in order to immobilize the antibody 35 on the outermost surface of the magnetic particle 36 for assay. As functional groups for binding the antibody 35 to the outermost surface of the magnetic particle 36 for assay, a carboxyl group, an amino group, a thiol group, an epoxy group, etc. are suitable. Accordingly, the second resin layer 34 is preferably a resin having such functional group.

The magnetic particle for assay according to this embodiment may further contain a fluorescent material. There is no limitation on a method of applying the fluorescent material, and a known technology may be selected. With regard to the timing of the application of the fluorescent material, for example, the fluorescent material may be mixed when the silica layer is formed, the fluorescent material may be applied after the formation of the silica layer and before the formation of the first resin layer, the fluorescent material may be mixed together when the first resin layer is formed, the fluorescent material may be applied after the formation of the first resin layer and before the formation of the second resin layer, or the fluorescent material may be applied after the formation of the second resin layer.

The method of applying the fluorescent material is achieved by, for example, forming the resin layer, and then mixing the fluorescent material and an organic solvent, and stirring the mixture under heating, and is also achieved by mixing the fluorescent material together when the resin layer is formed. There is no limitation on the fluorescent material to be selected, and a known fluorescent material may be selected. The fluorescent material may be non-polymerizable or polymerizable. Examples of the non-polymerizable fluorescent material include rhodamine B, rhodamine 6G, and Lumisis (trademark) E-400 and Lumisis (trademark) E-460 serving as europium complexes. Examples of the polymerizable fluorescent material include acryloxyethyl thiocarbamoyl rhodamine B and methacryloxyethyl thiocarbamoyl rhodamine B.

(Method of detecting Antigen using Magnetic Particle in Optical Waveguide-type Test System)

In an optical waveguide-type test system, when an antigen is detected from a sample such as a specimen, a magnetic particle to which an antibody that specifically binds to the antigen is bound, and a sensor on which an antibody that specifically binds to the antigen is immobilized are used. The sensor is arranged on a lower side in a direction of gravity, and an electromagnet, etc. that generates a magnetic force is arranged in a vicinity of the sensor. When the electromagnet is operated, the magnetic particle can be attracted to the vicinity of the sensor by the action of gravity and magnetic force. Then, through utilization of an antigen-antibody reaction, the magnetic particle attracted to the vicinity of the sensor binds to the antibody arranged on a sensor surface via the antigen. Further, when an electromagnet is arranged on an upper side in the direction of gravity, a magnetic particle which is not bound to the antibody arranged on the sensor surface can be pulled up in a direction opposite to gravity. Thus, the magnetic particle bound to the sensor and the magnetic particle free which is not thereto can be separated from each other.

The measurement of a change in amount of emitted light when light is caused to enter the sensor from a light source enables the measurement of the presence or absence, concentration, amount, etc. of the magnetic particle bound to the sensor surface via the antigen-antibody reaction. That is, when the magnetic particle bound to the sensor surface is present (its amount is large), the amount of emitted light is small as compared to that when the magnetic particle is absent (its amount is small). Accordingly, the presence or absence, concentration, amount, and etc. of a substance to be measured such as an antigen in the sample may be measured based on amount of attenuation from amount of emitted light detected before the magnetic particle binds to the sensor.

When the substance to be measured is detected by such method, it is important to reduce test errors by reducing non-specific adsorption of proteins other than the substance to be measured to the magnetic particle, or by reducing adsorption when there is no antigen on the sensor surface. For that purpose, a resin layer is formed on the surface of the magnetic particle. The resin layer preferably has a hydrophilic property, and the resin layer includes a functional group capable of binding a ligand such as an antibody.

[Second Embodiment]

The second embodiment of the present invention relates to a method of producing a magnetic particle. The method of producing a magnetic particle according to this embodiment aims to facilitate the control of the primary particle size and the secondary particle size.

The method of producing a magnetic particle according to the embodiment of the present invention is a method of producing a magnetic particle including a secondary particle in which a plurality of primary particles are aggregated, the method including: a step of obtaining an iron(III)-containing solution; a step of aging the iron(III)-containing solution; a step of obtaining a mixture in which the iron(III)-containing solution obtained in the step of aging and a precipitant are mixed; and a step of heating the mixture.

According to the production method according to this embodiment, the control of the primary particle size and the secondary particle size is easy, and hence a method of producing a magnetic particle having a wide range of magnetization (having a wide range of secondary particle sizes) in accordance with applications while being superparamagnetic can be provided.

The description is given below.

The method of producing a magnetic particle according to this embodiment is a method of producing a magnetic particle including a secondary particle in which a plurality of primary particles are aggregated. As described above, the magnetic properties of the $Fe_3O_4$ cluster depend on the primary and secondary particle sizes of the $Fe_3O_4$ cluster. The remanent magnetization of the $Fe_3O_4$ cluster decreases as the primary particle size decreases, and when the primary particle size becomes 20 nm or less, the remanent magnetization becomes zero, that is, so-called superparamagnetism is exhibited. In addition, the number of the primary particles to be aggregated, that is, the secondary particle size has a correlation with the magnetization of the $Fe_3O_4$ cluster, and the magnetization of the $Fe_3O_4$ cluster increases as the particle size increases. Accordingly, when the particle size of the $Fe_3O_4$ cluster can be controlled, a $Fe_3O_4$ cluster having a wide range of magnetization can be obtained. However, in this embodiment, a case in which the shape of the $Fe_3O_4$ cluster is deformed and bound or a case in which the shape becomes irregular is not included in the magnetic particle that is a secondary particle in which a plurality of primary particles are aggregated according to this embodiment. This is because, in such case, the secondary particles are in a state of being further associated, and can no longer be said to be the secondary particle in this embodiment.

<Synthesis of $Fe_3O_4$ Cluster>

A synthesis flow of the $Fe_3O_4$ cluster according to this embodiment is shown in Fig. 3. The synthesis of the $Fe_3O_4$ cluster mainly includes an iron(III)-containing solution preparation step (step of obtaining an iron(III)-containing solution), a mixture preparation step (step of obtaining a mixture), and a heating step (step of heating the mixture), and includes an aging step (step of aging the iron(III)-containing solution) as appropriate. Each step is described in detail below.

<Step of obtaining Iron(III)-containing Solution>

The method of producing a magnetic particle according to this embodiment includes a step of obtaining an iron(III)-containing solution containing ethylene glycol and iron(III) chloride anhydrous, and the iron(III)-containing solution obtained in the step of obtaining the iron(III)-containing solution preferably contains ethylene glycol and iron(III) chloride anhydrous. First, the preparation of the iron(III)-containing solution is described. The iron(III)-containing solution is prepared by dissolving an iron(III) compound in ethylene glycol. The iron(III) compound is suitably iron(III) chloride anhydrous or iron(III) chloride hexahydrate.

Those compounds may be used alone or as a mixture thereof at any ratio. In addition to iron(III) chloride, iron(III) nitrate, iron(III) acetate, iron(III) sulfate, ammonium iron(III) sulfate, iron(III) acetylacetonate, iron(III) hydroxide, iron(III) oxide, and hydrates thereof may be used alone or as a mixture thereof. The iron(III) compound is not limited thereto as long as the compound dissolves uniformly in ethylene glycol.

Ethylene glycol is a kind of organic compound called a polyol having a plurality of OH groups in its structure. The polyol is used for synthesis of metal fine particles (polyol method) and plays two roles of a solvent and a reducing agent. Ethylene glycol in this embodiment is also a solvent for dissolving iron(III) chloride, and at the same time, acts as a reducing agent for reducing part of Fe(III) to Fe(II). In addition to ethylene glycol, polyols that are liquid at normal temperature, such as diethylene glycol, triethylene glycol, 1,2-propanediol, and 1,3-propanediol, may be used, but those polyols cannot be used alone and are used by being mixed at any ratio with ethylene glycol.

$Fe_3O_4$ is an iron oxide containing Fe(II) and Fe(III) at a molar ratio of 1:2. When $Fe_3O_4$ is obtained by using ethylene glycol and iron(III) chloride, ethylene glycol serving as a reducing agent reduces part of Fe(III) to Fe(II), and hence $Fe_3O_4$ is produced. Accordingly, the concentration of iron(III) chloride in ethylene glycol serving as a solvent and a reducing agent is important. The concentration of iron(III) chloride is preferably 0.05 M or more and 0.2 M or less, more preferably 0.1 M or more. When the concentration of iron(III) chloride is less than 0.05 M, that is, when ethylene glycol serving as a reducing agent is excessive with respect to Fe(III), the reduction of Fe(III) is promoted to increase the amount of Fe(II), and hence $Fe_3O_4$ is not produced. Meanwhile, in the case where the concentration of iron(III) chloride is more than 0.2 M, when the concentration of the precipitant is increased in accordance with the concentration of iron(III) chloride, the viscosity of the mixture is increased. Accordingly, the preparation of a uniform mixture becomes difficult. In addition, convection in the mixture hardly occurs, and hence the mixture is hardly heated uniformly.

A case where the method of producing a magnetic particle according to this embodiment is a method of producing a magnetic particle including a secondary particle in which a plurality of primary particles are aggregated, the method including: a step of obtaining an iron(III)-containing solution; a step of aging the iron(III)-containing solution; a step of obtaining a mixture in which the iron(III)-containing solution obtained in the step of aging and a precipitant are mixed; and a step of heating the mixture, is described below. In the method of producing a magnetic particle according to this embodiment, the iron(III)-containing solution obtained in the step of obtaining the iron(III)-containing solution may contain ethylene glycol and iron(III) chloride anhydrous. In addition, in the method of producing a magnetic particle according to this embodiment, the iron(III)-containing solution obtained in the step of obtaining the iron(III)-containing solution may contain ethylene glycol, iron(III) chloride anhydrous, and externally added water. In addition, in the method of producing a magnetic particle according to this embodiment, the iron(III)-containing solution obtained in the step of obtaining the iron(III)-containing solution may contain ethylene glycol, iron(III) chloride anhydrous, and iron(III) chloride hexahydrate. In addition, in the method of producing a magnetic particle according to this embodiment, the iron(III)-containing solution obtained in the step of obtaining the iron(III)-containing solution may contain ethylene glycol, iron(III) chloride hexahydrate, and externally added water.

<Step of obtaining Mixture>

The method of producing a magnetic particle according to this embodiment includes a step of obtaining a mixture in which the iron(III)-containing solution and a precipitant are mixed. In addition, when the method of producing a magnetic particle according to this embodiment includes an aging step, the method includes a step of obtaining a mixture in which the iron(III)-containing solution obtained in the step of aging and a precipitant are mixed. When the precipitant is added to the iron(III)-containing solution, precursor particles precipitate. A dispersion liquid in which the precursor particles are dispersed is called a mixture. The precursor particle is a particle of an amorphous and watersoluble iron(III) compound. In the method of producing a magnetic particle according to this embodiment, the precipitant is an acetate preferably. Specifically, urea, an alkali metal hydroxide, and an acetate may each be used as the precipitant, and an acetate, such as sodium acetate, potassium acetate, or ammonium acetate, is particularly preferred.

<Water in Mixture>

In the method of producing a magnetic particle according to this embodiment, water derived from at least one of iron(III) chloride hexahydrate or externally added water is added in at least one of the step of obtaining the iron(III)-containing solution or the step of obtaining the mixture. Water may be added to the mixture. Presence of water in the mixture affects the primary particle size and shape of the $Fe_3O_4$ cluster to be produced. Water may be added to ethylene glycol before the dissolution of the iron(III) compound, may be added to the iron(III)-containing solution, or may be added to the mixture after the addition of the precipitant.

Water in the mixture may be hydration water of a hydrate such as iron(III) chloride hexahydrate and intentionally added water (hereinafter also referred to as "externally added water"). Water derived from at least one of iron(III) chloride hexahydrate or externally added water is required to be present. In the case where water is absent in the mixture, for example, the iron(III) compound is iron(III) chloride anhydrous, when a mixture formed of ethylene glycol, iron(III) chloride anhydrous, and sodium acetate is heated, $Fe_3O_4$ clusters having small primary particle sizes are produced, but the $Fe_3O_4$ clusters are deformed or the $Fe_3O_4$ clusters bind to each other.

When a molar ratio between iron(III) chloride anhydrous and iron(III) chloride hexahydrate is changed while the concentration of iron(III) chloride is kept constant, as the ratio of iron(III) chloride hexahydrate increases, that is, as the amount of water in the mixture increases, the primary particle size of the $Fe_3O_4$ cluster increases, but the deformation and binding of the cluster are suppressed and its shape becomes spherical. Also when externally added water is intentionally added to a mixture containing ethylene glycol and iron(III) chloride anhydrous, an increase in particle size of each of the primary particles of the $Fe_3O_4$ cluster and the spheroidization of the secondary particle thereof described above are observed with an increase in addition amount of the externally added water.

The reason why the primary particle size of the $Fe_3O_4$ cluster increases with an increase in amount of water in the mixture is presumed to be that the solubility of $Fe_3O_4$ in water is higher than the solubility thereof in ethylene glycol, and hence a degree of supersaturation of $Fe_3O_4$ becomes smaller and the number of nuclei to be produced decreases when water is present in the mixture. Meanwhile, the shape of the $Fe_3O_4$ cluster may be affected by the interfacial free energy between $Fe_3O_4$ and the solvent. The interfacial free energy between $Fe_3O_4$ and ethylene glycol is different from the interfacial free energy between $Fe_3O_4$ and an ethylene glycol/water mixed solvent, and hence the shape of the $Fe_3O_4$ cluster may change depending on the presence or absence of water in the mixture.

In the method of producing a magnetic particle according to this embodiment, amount of water per 30 mL of ethylene glycol in the iron(III)-containing solution is preferably more than 0 mL and 10.0 mL or less, more preferably 0.2 mL or more and 8.0 mL or less. When water is not incorporated at all, there is no effect on the spheroidization of the $Fe_3O_4$ cluster. When the amount of water is more than 10.0 mL, the primary particles of $Fe_3O_4$ do not form a cluster. As described above, the primary particles of the $Fe_3O_4$ cluster increase in particle size as the amount of water increases, but when the amount of water is 8.0 mL or less, the increase in particle size of the primary particles can be suppressed by combination with operations, such as the addition of additives, stirring during heating, and aging to be described later. In addition, when the amount of water is more than 10.0 mL, $Fe_2O_3$ is produced in addition to $Fe_3O_4$. The amount of water per 30 mL of ethylene glycol is the sum of hydration water of the hydrate and externally added water. For example, when 0.9 mmol of iron(III) chloride anhydrous and 3.6 mmol of iron(III) chloride hexahydrate are dissolved in 30 mL of ethylene glycol, and 0.4 mL of externally added water is added, the content of the hydration water in 3.6 mmol of iron(III) chloride hexahydrate is 0.39 mL, and hence the amount of water per 30 mL of ethylene glycol is 0.79 mL.

<Additive in Mixture>

In order to reduce the particle size of each of primary particles serving as units for forming the $Fe_3O_4$ cluster and to improve the dispersibility of the $Fe_3O_4$ cluster, an additive may be mixed in the mixture. A high molecular compound, a low molecular compound, a surfactant, a chelating agent, etc. may each be used as the additive, but an additive having a group that becomes a carboxylate ion ($-COO^-$) in ethylene glycol is preferred. The carboxylate ion ($-COO^-$) coordinates strongly to Fe(III), and hence the growth rate of the primary particles of $Fe_3O_4$ can be controlled. A carboxylic acid and a carboxylic acid salt may be used as the additive having a group that becomes a carboxylate ion ($-COO^-$) in ethylene glycol, but a carboxylic acid salt that has a high degree of dissociation and easily generates a carboxylate ion is suitably used. Specifically, citric acid, malic acid, acrylic acid, tartaric acid, lactic acid, oleic acid, succinic acid, maleic acid, fumaric acid, and alkali metal salts thereof are preferred, and trisodium citrate and sodium acrylate are particularly preferred.

Those additives each have such spheroidizing effect on the $Fe_3O_4$ cluster as described above in addition to the reducing effect on the particle size of each of the primary particles of the $Fe_3O_4$ cluster. That is, even in a case where water is absent in the mixture, when the additive coexists, a spherical $Fe_3O_4$ cluster can be obtained. In addition, those additives each have a reducing effect on the particle size of the secondary particle.

<Step of heating Mixture>

The method of producing a magnetic particle according to this embodiment includes a step of heating the mixture. The heating temperature of the mixture is 160°C or more and 240°C or less, preferably 180°C or more and 220°C or less. When the heating temperature is less than 160°C, $Fe_3O_4$ is not produced. Meanwhile, when the heating temperature is more than 240°C, a reaction rate is high and the particle size distribution of the $Fe_3O_4$ cluster becomes large.

The heating time of the mixture is 2 hours or more and 24 hours or less, preferably 4 hours or more and 12 hours or less. When the heating time is less than 2 hours, conversion from the precursor particles to the $Fe_3O_4$ cluster is not completed, and when the heating time is more than 24 hours, the primary particle size of the $Fe_3O_4$ cluster increases owing to Ostwald ripening.

The mixture may contain a component having a boiling point equal to or lower than the heating temperature, such as ethylene glycol having a boiling point of 197°C or water having a boiling point of 100°C. Accordingly, it is preferred to use a pressure-resistant closed vessel which is generally called an "autoclave" in the heating of the mixture.

In addition, the mixture may be heated under a stirring condition through use of a vessel provided with a stirring function. In the case of the heating under a static condition using the closed vessel described above, as the reaction proceeds, a concentration gradient occurs in the mixture, and the mixture becomes nonuniform. Meanwhile, in the case of the heating under the stirring condition, mass transfer is promoted and the mixture can always maintain a uniform state, and hence the heating is effective in reducing the particle size of each of the primary particles.

After the heating, the vessel is left at room temperature until the vessel is sufficiently cooled, and then the contents are taken out. The resultant $Fe_3O_4$ cluster is washed twice with ethanol and twice with water, and is dried in a dry oven at 80°C.

<Step of aging Iron(III)-containing Solution>

The method of producing a magnetic particle according to this embodiment may include a step of aging the iron(III)-containing solution. The iron(III)-containing solution is a solution in which the iron(III) compound is completely dissolved in ethylene glycol to be brought into a uniform state. The aging is an operation of holding this iron(III)-containing solution at a certain temperature for a certain time period.

When the aging is performed, for example, a reaction in which a ligand of Fe(III) exchanges from a chloride ion to ethylene glycol is presumed to be promoted, and hence solvation state of Fe(III) is presumed to change and the change is presumed to affect the structure of the $Fe_3O_4$ cluster serving as a final product. In addition, while the iron(III) compound macroscopically appears to be completely dissolved, microscopically, the iron(III) compound may be dispersed in ethylene glycol in a state like a kind of oligomer, and the oligomer is turned into a monomer by the aging, and hence the activity of the dissolved species of Fe(III) increases, and as a result, the primary and secondary particle sizes of the $Fe_3O_4$ cluster change.

Thus, the aging is related to the dissolved species of Fe(III), and hence the timing of the aging is essential to be done before the addition of the precipitant to the iron(III)-containing solution. Even when the aging is performed after the precipitant has been added to the iron(III)-containing solution to produce precursor particles, the structure of the $Fe_3O_4$ cluster does not change.

As aging temperature increases and as aging time increases, the primary particle size of the $Fe_3O_4$ cluster decreases and the secondary particle size thereof increases. In the method of producing a magnetic particle according to this embodiment, the aging temperature in the step of aging is preferably 0°C or more and 130°C or less, more preferably 10°C or more and 130°C or less. When the aging temperature is less than 0°C, the effect of the aging is small, and when the aging temperature is more than 130°C, the primary particles of $Fe_3O_4$ aggregate irregularly and do not form a spherical secondary particle. In the method of producing a magnetic particle according to this embodiment, the aging time in the step of aging is preferably 1 hour or more and 500 hours or less, more preferably 1 hour or more and 200 hours or less. The aging for less than 1 hour does not affect the structure of the $Fe_3O_4$ cluster, and the aging for more than 500 hours is excessive because the effect saturates in a certain time period.

When the iron(III)-containing solution is aged, the timing of the addition of an additive that affects the primary and secondary particle sizes of the $Fe_3O_4$ cluster is also important. Even when the kind and concentration of the additive are same, in particular, the secondary particle size changes dramatically depending on the timing of the addition of the additive.

When the additive is added after the aging of the iron(III)-containing solution, the particle size-reducing effect of the additive increases, and the secondary particle size decreases as compared to that when the additive is added to the iron(III)-containing solution without aging. Meanwhile, when the additive is added before the aging of the iron(III)-containing solution, the particle size-reducing effect of the additive is offset by the particle size-increasing effect of the aging, and the secondary particle size increases as compared to that when the additive is added to the iron(III)-containing solution without aging.

In addition, when the concentration of the additive to be added before the aging of the iron(III)-containing solution is a low concentration of 0.01 M or less, the particle size-increasing effect of the aging is further promoted by the additive, and the secondary particle becomes huge. This is conceived to be because the structure of the dissolved species of Fe(III) is changed by the aging in the coexistence of the additive.

As described above, the secondary particle size of the $Fe_3O_4$ cluster obtained by using the iron(III)-containing solution after the aging depends on a degree of the aging. A secondary particle of 500 nm or more and 3,000 nm or less can be produced. When two or more kinds of iron(III)-containing solutions having different degrees of aging are used as a mixture thereof at any ratio, the secondary particle size of the $Fe_3O_4$ cluster can be controlled. Such method of controlling the particle size of the $Fe_3O_4$ cluster is called a blend method. A synthesis flow of the $Fe_3O_4$ cluster by the blend method is shown in Fig. 4.

For example, in a case where the iron(III)-containing solution having a weak degree of aging and the iron(III)-containing solution having a strong degree of aging are an iron(III)-containing solution having a short aging time and capable of providing a $Fe_3O_4$ cluster having a particle size of 500 nm, and an iron(III)-containing solution having a long aging time and capable of providing a $Fe_3O_4$ cluster having a particle size of 1,000 nm, respectively, when those solutions are used as a mixture thereof at any ratio, the particle size of the $Fe_3O_4$ cluster to be produced can be controlled in the range of 500 nm or more and 1,000 nm or less.

<Method of identifying Product>

Product identification was performed by X-ray diffraction measurement (XRD). The XRD was performed by using X'PertPro manufactured by Spectris Co., Ltd. under conditions of θ-2θ scan, a tube voltage of 40 kV, a tube current of 45 mA, a scanning angle of from 10° to 70°, a step of 0.02°, and a measurement time/step of 1 sec.

<Method of measuring Primary Particle Size of $Fe_3O_4$ Cluster>

The primary particle size of the $Fe_3O_4$ cluster was measured by scanning electron microscope measurement (SEM) or XRD.
The SEM was performed by using S-4800 manufactured by Hitachi High-Technologies Corporation under conditions of an acceleration voltage of 5 kV and an irradiation current of 10 μA. Through use of an SEM image at a magnification of 100,000, particle sizes of 50 primary particles randomly sampled from the image were measured, and an average value thereof was calculated.
The XRD was performed by using X'PertPro manufactured by Spectris Co., Ltd. under conditions of θ-2θ scan, a tube voltage of 40 kV, a tube current of 45 mA, a scanning angle of from 33° to 38°, a step of 0.01°, and a measurement time/step of 2 sec, and a particle size D nm of each of the primary particles was calculated by using Scherrer equation (1):

$$D = K\lambda/\beta\cos\theta \qquad (1)$$

where K represents Scherrer constant (K=0.89), λ represents a wavelength of an X-ray (λ=0.1542 nm), β represents half-width rad of diffraction peak of (311) plane of $Fe_3O_4$, and θ represents Bragg angle (0.3091 rad).

<Method of measuring Secondary Particle Size of $Fe_3O_4$ Cluster>

The secondary particle size of the $Fe_3O_4$ cluster was measured by using SEM or dynamic light scattering (DLS).
The SEM was performed by using the above-mentioned apparatus and conditions. Through use of an SEM image at a magnification of 10,000, particle sizes of 50 secondary particles randomly sampled from the image were measured, and an average value thereof was calculated.
A particle size distribution of the $Fe_3O_4$ cluster by the DLS was obtained by using a laser diffraction/scattering particle size distribution measuring apparatus Partica LA-950 manufactured by Horiba, Ltd. Median diameters D50 and D90 were calculated from measured number-based particle size distribution.

<Method of measuring Magnetic Properties of $Fe_3O_4$ Cluster>

Magnetic properties of the $Fe_3O_4$ cluster were measured by measuring a hysteresis curve through use of magnetic property measurement system MPMS3 manufactured by Quantum Design, Inc. at measurement temperature of 298 K, in a VSM mode, and in a magnetic field range from -20,000 Oe to +20,000 Oe.

<Production of Magnetic Particle for Assay>

The $Fe_3O_4$ cluster produced by controlling the particle size may be used as a magnetic particle for assay by further forming an inorganic layer or an organic layer on the particle surface. As the magnetic particle for assay, first, in order to secure the surface smoothness of the $Fe_3O_4$ cluster, a silica layer may be formed by a wet method such as Stober method. In addition, it is preferred to form a styrene-based resin layer having high chemical stability and high hydrophobicity as a first resin layer, and further form a polyglycidyl methacrylate layer as a second resin layer. A ligand can bind to the surface of the second resin layer preferably, and the ligand be an antibody preferably. As functional groups for binding an antibody to the outermost surface of the magnetic particle for assay, a carboxyl group, an amino group, a thiol group, an epoxy group, etc. are suitable. Accordingly, the second resin layer is a resin having such functional group preferably.

The magnetic particle for assay may further contain a fluorescent material. There is no limitation on the method of applying the fluorescent material, and a known technology may be selected. As the timing of the application of the fluorescent material, for example, the fluorescent material may be mixed at the time of the formation of the silica layer, the fluorescent material may be applied after the formation of the silica layer and before the formation of the first resin layer, the fluorescent material may be mixed together at the time of the formation of the first resin layer, the fluorescent material may be applied after the formation of the first resin layer and before the formation of the second resin layer, or the fluorescent material may be applied after the formation of the second resin layer. The method of applying the fluorescent material is achieved by, for example, forming the resin layer, and then mixing the fluorescent material and an organic solvent, and stirring the mixture under heating, and is also achieved by mixing the fluorescent material together at the time of the formation of the resin layer. There is no limitation on the fluorescent material to be selected, and a known fluorescent material may be selected. The fluorescent material may be non-polymerizable or polymerizable. Examples of the non-polymerizable fluorescent material include rhodamine B, rhodamine 6G, and Lumisis (trademark) E-400 and Lumisis (trademark) E-460 serving as europium complexes, and examples of the polymerizable fluorescent material include acryloxyethyl thiocarbamoyl rhodamine B and methacryloxyethyl thiocarbamoyl rhodamine B.

[Examples]

The present invention is described in more detail by way of Examples. However, the present invention is not limited to the following Examples. In addition, production conditions of Examples and results of $Fe_3O_4$ clusters are summarized in Table 1.

<<Synthesis of $Fe_3O_4$ Cluster>>

<Example 1>

4.5 Millimoles of iron(III) chloride anhydrous ($FeCl_3$; Kishida Chemical Co., Ltd.) was dissolved in 30 mL of ethylene glycol (dehydrated; FUJIFILM Wako Pure Chemical Corporation) to prepare a 0.15 M ethylene glycol solution of iron(III) chloride (iron(III)-containing solution).

After 0.4 mL of externally added $H_2O$ had been added to the iron(III)-containing solution, aging was performed. Aging temperature was set to 25°C, and aging time was set to 72 hours. In addition, the aging was performed in a thermostat after the iron(III)-containing solution had been injected into a pressure-resistant vessel including a Teflon (trademark) inner cylinder and the vessel had been tightly sealed. To the iron(III)-containing solution after the aging, 2.7 g of sodium acetate ($CH_3COONa$; Kishida Chemical Co., Ltd.) was added to provide a mixture in which precursor particles were dispersed. The mixture was injected into a pressure-resistant vessel including a Teflon (trademark) inner cylinder and the vessel was tightly sealed. The pressure-resistant vessel was heated in an oven at 180°C for 12 hours. After the completion of the reaction, the pressure-resistant vessel was sufficiently cooled to around room temperature, and then the produced $Fe_3O_4$ cluster was taken out. The resultant $Fe_3O_4$ cluster was washed twice with ethanol and twice with water, and was dried in an oven at 80°C. SEM images of the produced $Fe_3O_4$ cluster are shown in Fig. 5A and Fig. 5B.

<Example 2>

A magnetic particle was produced by the same procedure as that of Example 1 except that, in Example 1, the aging temperature of the iron(III)-containing solution was set to 60°C, and the aging time was set to 72 hours. SEM images of the produced $Fe_3O_4$ cluster are shown in Fig. 6A and Fig. 6B.

<Example 3>

A magnetic particle was produced by the same procedure as that of Example 1 except that, in Example 1, the aging temperature of the iron(III)-containing solution was set to 100°C, and the aging time was set to 72 hours.

<Example 4>

A magnetic particle was produced by the same procedure as that of Example 1 except that, in Example 1, the aging temperature of the iron(III)-containing solution was set to 60°C, and the aging time was set to 24 hours.

<Example 5>

A magnetic particle was produced by the same procedure as that of Example 1 except that, in Example 1, the aging temperature of the iron(III)-containing solution was set to 60°C, and the aging time was set to 120 hours.

<Example 6>

A magnetic particle was produced by the same procedure as that of Example 1 except that, in Example 1, the aging temperature of the iron(III)-containing solution was set to 60°C, and the aging time was set to 168 hours. SEM images of the produced $Fe_3O_4$ cluster are shown in Fig. 7A and Fig. 7B.

<Example 7>

In Example 1, the aging temperature of the iron(III)-containing solution was set to 60°C, and the aging time was set to 168 hours. Trisodium citrate was added to the iron(III)-containing solution after the aging so as to have a concentration of 0.01 M. A magnetic particle was produced by the same procedure as that of Example 1 except for those conditions.

<Example 8>

In Example 1, a solution obtained by dissolving 0.9 mmol of $FeCl_3$ and 3.6 mmol of $FeCl_3 \cdot 6H_2O$ in ethylene glycol was used as the iron(III)-containing solution. The addition amount of externally added $H_2O$ was set to 0 mL. The aging temperature of the iron(III)-containing solution was set to 60°C, and the aging time was set to 168 hours. A magnetic particle was produced by the same procedure as that of Example 1 except for those conditions. SEM images of the produced $Fe_3O_4$ cluster are shown in Fig. 8A and Fig. 8B.

<Example 9>

In Example 1, a solution obtained by dissolving 0.9 mmol of $FeCl_3$ and 3.6 mmol of $FeCl_3 \cdot 6H_2O$ in ethylene glycol was used as the iron(III)-containing solution. The aging temperature of the iron(III)-containing solution was set to 25°C, and the aging time was set to 168 hours. A magnetic particle was produced by same procedure as that of Example 1 except for those conditions.

<Example 10>

In Example 1, a solution obtained by dissolving 0.9 mmol of $FeCl_3$ and 3.6 mmol of $FeCl_3 \cdot 6H_2O$ in ethylene glycol was used as the iron(III)-containing solution. The addition amount of externally added $H_2O$ was set to 0 mL. The aging temperature of the iron(III)-containing solution was set to 60°C, and the aging time was set to 168 hours. Trisodium citrate was added to the iron(III)-containing solution after the aging so as to have a concentration of 0.01 M. A magnetic particle was produced by the same procedure as that of Example 1 except for those conditions.

<Example 11>

In Example 1, a solution obtained by dissolving 3.6 mmol of $FeCl_3$ and 0.9 mmol of $FeCl_3 \cdot 6H_2O$ in ethylene glycol was used as the iron(III)-containing solution. The aging temperature of the iron(III)-containing solution was set to 60°C, and the aging time was set to 168 hours. Sodium acrylate was added to the iron(III)-containing solution after the aging so as to have a concentration of 0.01 M. A magnetic particle was produced by the same procedure as that of Example 1 except for those conditions.

<Example 12>

In Example 1, a solution obtained by dissolving 4.5 mmol of $FeCl_3 \cdot 6H_2O$ in ethylene glycol was used as the iron(III)-containing solution. The addition amount of externally added $H_2O$ was set to 0 mL. A magnetic particle was produced by the same procedure as that of Example 1 except for those conditions.

<Example 13>

In Example 1, a solution obtained by dissolving 4.5 mmol of $FeCl_3 \cdot 6H_2O$ in ethylene glycol was used as the iron(III)-containing solution. The aging temperature of the iron(III)-containing solution was set to 60°C, and the aging time was set to 168 hours. A magnetic particle was produced by the same procedure as that of Example 1 except for those conditions.

<Example 14>

In Example 1, a solution obtained by dissolving 4.5 mmol of $FeCl_3 \cdot 6H_2O$ in ethylene glycol was used as the iron(III)-containing solution. Sodium acrylate was added to the iron(III)-containing solution before the aging so as to have a concentration of 0.01 M. The aging temperature of the iron(III)-containing solution was set to 60°C, and the aging time was set to 168 hours. A magnetic particle was produced by the same procedure as that of Example 1 except for those conditions. SEM images of the produced $Fe_3O_4$ cluster are shown in Fig. 9A and Fig. 9B.

<Example 15>

In Example 1, the aging temperature of the iron(III)-containing solution was set to 60°C, and the aging time was set to 168 hours. The mixture was heated under a stirring condition through use of a flask and a mechanical stirrer. A magnetic particle was produced by the same procedure as that of Example 1 except for those conditions.

<Example 16>

In Example 1, a solution obtained by dissolving 4.5 mmol of $FeCl_3 \cdot 6H_2O$ in ethylene glycol was used as the iron(III)-containing solution. The addition amount of externally added $H_2O$ was set to 0 mL. Disodium malate was added to the iron(III)-containing solution before the aging so as to have a concentration of 0.01 M. The aging temperature of the iron(III)-containing solution was set to 60°C, and the aging time was set to 168 hours. A magnetic particle was produced by the same procedure as that of Example 1 except for those conditions.

<Example 17>

In Example 1, a solution obtained by dissolving 4.5 mmol of $FeCl_3 \cdot 6H_2O$ in ethylene glycol was used as the iron(III)-containing solution. The addition amount of externally added $H_2O$ was set to 0 mL. Disodium malate was added to the iron(III)-containing solution before the aging so as to have a concentration of 0.08 M. The aging temperature of the iron(III)-containing solution was set to 60°C, and the aging time was set to 168 hours. A magnetic particle was produced by the same procedure as that of Example 1 except for those conditions.

<Example 18>

In Example 1, a solution obtained by dissolving 4.5 mmol of $FeCl_3 \cdot 6H_2O$ in ethylene glycol was used as the iron(III)-containing solution. The addition amount of externally added $H_2O$ was set to 0 mL. Sodium oleate was added to the iron(III)-containing solution before the aging so as to have a concentration of 0.01 M. The aging temperature of the iron(III)-containing solution was set to 60°C, and the aging time was set to 168 hours. A magnetic particle was produced by the same procedure as that of Example 1 except for those conditions.

<Example 19>

In Example 1, a solution obtained by dissolving 4.5 mmol of $FeCl_3 \cdot 6H_2O$ in ethylene glycol was used as the iron(III)-containing solution. The addition amount of externally added $H_2O$ was set to 0 mL. Sodium oleate was added to the iron(III)-containing solution before the aging so as to have a concentration of 0.08 M. The aging temperature of the iron(III)-containing solution was set to 60°C, and the aging time was set to 168 hours. A magnetic particle was produced by the same procedure as that of Example 1 except for those conditions.

<Example 20>

In Example 1, sodium acrylate was added to the iron(III)-containing solution before the aging so as to have a concentration of 0.04 M. The aging temperature of the iron(III)-containing solution was set to 60°C, and the aging time was set to 168 hours. A magnetic particle was produced by the same procedure as that of Example 1 except for those conditions.

<Example 21>

In Example 1, the aging temperature of the iron(III)-containing solution was set to 60°C, and the aging time was set to 168 hours. Sodium acrylate was added to the iron(III)-containing solution after the aging so as to have a concentration of 0.04 M. A magnetic particle was produced by the same procedure as that of Example 1 except for those conditions.

<Examples 22 to 29>

In Example 1, a solution obtained by dissolving 4.5 mmol of $FeCl_3 \cdot 6H_2O$ in ethylene glycol was used as the iron(III)-containing solution. The addition amount of externally added $H_2O$ was set to 0 mL. The aging temperature of the iron(III)-containing solution was set to 60°C, and the aging time was set to from 4 hours to 168 hours. Magnetic particles were each produced by the same procedure as that of Example 1 except for those conditions. With regard to Examples 22 to 29, SEM images of the produced $Fe_3O_4$ clusters are shown in Fig. 10A to Fig. 10H.

<Comparative Example 1>

A magnetic particle was produced by the same procedure as that of Example 1 except that, in Example 1, the aging was not performed on the iron(III)-containing solution.

<Comparative Example 2>

A magnetic particle was produced by the same procedure as that of Example 1 except that, in Example 1, the addition amount of externally added $H_2O$ was set to 0 mL. SEM images of the produced $Fe_3O_4$ cluster are shown in Fig. 11A and Fig. 11B.

<Comparative Example 3>

In Example 1, the addition amount of externally added $H_2O$ was set to 8.5 mL. The aging temperature of the iron(III)-containing solution was set to 60°C, and the aging time was set to 168 hours. A magnetic particle was produced by the same procedure as that of Example 1 except for those conditions. SEM images of the produced $Fe_3O_4$ cluster are shown in Fig. 12A and Fig. 12B. The formation of the $Fe_3O_4$ cluster was not able to be observed.

<Comparative Example 4>

A magnetic particle was produced by the same procedure as that of Example 1 except that, in Example 1, the aging temperature of the iron(III)-containing solution was set to 140°C.

<Comparative Example 5>

In Example 1, a solution obtained by dissolving 4.5 mmol of $FeCl_3 \cdot 6H_2O$ in 1,2-propanediol (Kishida Chemical Co., Ltd.) was used as the iron(III)-containing solution. The aging temperature of the iron(III)-containing solution was set to 60°C, and the aging time was set to 168 hours. A magnetic particle was produced by the same procedure as that of Example 1 except for those conditions.

<Comparative Example 6>

In Example 1, the aging was not performed on the iron(III)-containing solution. The mixture was heated under a stirring condition through use of a flask and a mechanical stirrer. A magnetic particle was produced by the same procedure as that of Example 1 except for those conditions.

<<Particle Size Control of $Fe_3O_4$ Cluster by Blend Method>>

<Examples 30 to 32>

A solution A (weak aging solution), which was a 0.15 M iron(III)-containing solution obtained by dissolving iron(III) chloride hexahydrate in ethylene glycol, and a solution B (strong aging solution), which was obtained by aging a 0.15 M iron(III)-containing solution obtained by dissolving iron(III) chloride hexahydrate in ethylene glycol at 60°C for 168 hours, were prepared.

The solution A and the solution B described above were mixed at any ratio to prepare 30 mL of a solution C. The volume ratio "solution A:solution B" was changed in the range of from 100:0 to 0:100. A case in which the volume ratio "solution A:solution B" was set to 100:0 was adopted as Synthesis Example 1, and a case in which the volume ratio "solution A:solution B" was set to 0:100 was adopted as Synthesis Example 2.

To the prepared solution C, 2.7 g of $CH_3COONa$ was added to provide a mixture in which precursor particles were dispersed. The mixture was injected into a pressure-resistant vessel including a Teflon (trademark) inner cylinder and the vessel was tightly sealed. The pressure-resistant vessel was heated in an oven at 180°C for 12 hours. After the completion of the reaction, the pressure-resistant vessel was sufficiently cooled to around room temperature, and then the produced $Fe_3O_4$ cluster was taken out. The resultant $Fe_3O_4$ cluster was washed twice with ethanol and twice with water, and was dried in an oven at 80°C.

The production conditions of Examples and the results of the $Fe_3O_4$ clusters are summarized in Table 2. SEM images of the produced $Fe_3O_4$ clusters are shown in Fig. 13A to Fig. 13E.

<<Identification of Product>>

As a result of XRD, the particles each had a single phase of $Fe_3O_4$. XRD patterns of the particles produced in Examples 22, 24, 26, and 28 are shown in Fig. 14.

<<Measurement of Magnetic Properties of $Fe_3O_4$ Cluster>>

As a result of the measurement of magnetic properties, the particles each showed superparamagnetic behavior having no hysteresis characteristics. Hysteresis curves of Examples 22, 24, 26, and 28 are shown in Fig. 15A to Fig. 15D, respectively.

<<Production of Magnetic Particle for Assay>>

<Reference Example 1>

0.5 Gram of the magnetic particle produced in Example 1 was dispersed in a mixed solution containing 75 mL of ethanol (manufactured by Kishida Chemical Co., Ltd.) and 75 mL of pure water. Next, 1.5 mL of tetraethyl orthosilicate (manufactured by Kishida Chemical Co., Ltd.) was added, 22.5 mL of 28% ammonia water (manufactured by Kishida Chemical Co., Ltd.) was added as a catalyst, and the mixture was subjected to a reaction for 1.5 hours while being stirred. After the reaction, the solvent was removed by centrifugation, and the residue was washed seven times with pure water. Thus, a silica layer was formed on the magnetic particle.

The resultant silica-coated magnetic particle was dispersed in a mixed solution containing 10 mL of ethanol and 10 mL of pure water, and 100 μL of 3-methacryloxypropyltrimethoxysilane (LS-3380, manufactured by Shin-Etsu Chemical Co., Ltd.) was added as a silane coupling agent and mixed sufficiently. Next, 2 mL of 28% ammonia water was added and the mixture was stirred for 1.5 hours. Next, the magnetic particle was collected with a magnet so that the solvent was removed. The magnetic particle was washed sufficiently with pure water, and then 60 mL of nitrogen-bubbled pure water was added to provide a water dispersion liquid.

Next, the dispersion liquid was loaded into a 200 mL four-necked flask, and was stirred for 15 minutes while being stirred at stirring speed of 200 rpm with nitrogen bubbling. Subsequently, after the nitrogen bubbling had been switched to nitrogen flow, 50 μL of styrene (manufactured by Kishida Chemical Co., Ltd.) was added to the dispersion liquid. Next, 0.02 g of potassium persulfate (manufactured by Sigma-Aldrich) was dissolved in 2 mL of pure water degassed by nitrogen bubbling in advance, and 1 mL of the solution was added into the flask. Next, through use of an oil bath, the mixture was heated at 35°C for 30 minutes, and then the temperature was raised to 60°C and held for 1 hour. Subsequently, 200 μL of glycidyl methacrylate (manufactured by Kishida Chemical Co., Ltd.) was added, and the mixture was further held for 12 hours to complete polymerization. After the completion of the polymerization, the solvent was removed by centrifugation, and the magnetic particle was washed sufficiently with pure water.

Subsequently, 10 mg of the resultant magnetic particle was dispersed in 5 mL of pure water. Separately from the dispersion liquid, 350 mg of mercaptosuccinic acid (manufactured by Kishida Chemical Co., Ltd.) was dissolved in 5 mL of pure water, and 0.8 mL of triethylamine (manufactured by Tokyo Chemical Industry Co., Ltd.) was added for pH adjustment. 1 Milliliter of the solution was added to the particle dispersion liquid, and the mixture was stirred sufficiently and subjected to heat treatment at 60°C for 3 hours. After that, the solvent was removed by centrifugation, and the magnetic particle was washed sufficiently with pure water. Thus, a dispersion liquid of the magnetic particle including a silica layer, a first resin layer, and a second resin layer was obtained.

<Reference Example 2>

0.5 Gram of the magnetic particle produced in Example 1 was dispersed in a mixed solution containing 75 mL of ethanol and 75 mL of pure water. Next, 1.5 mL of tetraethyl orthosilicate was added, 22.5 mL of 28% ammonia water was added as a catalyst, and the mixture was subjected to a reaction for 1.5 hours while being stirred. After the reaction, the solvent was removed by centrifugation, and the magnetic particle was washed seven times with pure water. Thus, a silica layer was formed on the magnetic particle.

The resultant silica-coated magnetic particle was dispersed in a mixed solution containing 10 mL of ethanol and 10 mL of pure water, and 100 μL of 3-methacryloxypropyltrimethoxysilane was added as a silane coupling agent and mixed sufficiently. Next, 2 mL of 28% ammonia water was added and the mixture was stirred for 1.5 hours. Next, the magnetic particle was collected with a magnet so that the solvent was removed. The magnetic particle was washed sufficiently with pure water, and then 60 mL of nitrogen-bubbled pure water was added to provide a water dispersion liquid.

Next, the dispersion liquid was loaded into a 200 mL four-necked flask, and was stirred for 15 minutes while being stirred at stirring speed of 200 rpm with nitrogen bubbling. Subsequently, after the nitrogen bubbling had been switched to nitrogen flow, 50 μL of styrene in which 5 mg of Lumisis (trademark) E-400 (manufactured by Central Techno Corporation) serving as a europium complex, 2 mg of sodium styrenesulfonate (manufactured by Tokyo Chemical Industry Co., Ltd.), and 5 mg of divinylbenzene (manufactured by Tokyo Chemical Industry Co., Ltd.) were dissolved was added to the dispersion liquid.

Next, through use of an oil bath, the mixture was heated at 35°C for 30 minutes. After that, 1 mg of 2,2'-azobis(2-methylpropionamidine) dihydrochloride (manufactured by FUJIFILM Wako Pure Chemical Corporation) was dissolved in 1 mL of pure water degassed by nitrogen bubbling in advance, and 500 μL of the solution was added into the flask. The temperature was raised to 60°C and held for 2 hours. Subsequently, 200 μL of glycidyl methacrylate was added, and the mixture was further held for 12 hours to complete polymerization. After the completion of the polymerization, the solvent was removed by centrifugation, and the magnetic particle was washed sufficiently with pure water.

Subsequently, 10 mg of the resultant magnetic particle was dispersed in 5 mL of pure water. Separately from the dispersion liquid, 350 mg of mercaptosuccinic acid was dissolved in 5 mL of pure water, and 0.8 mL of triethylamine was added for pH adjustment. 1 Milliliter of the solution was added to the particle dispersion liquid, and the mixture was stirred sufficiently and subjected to heat treatment at 60°C for 3 hours. After that, the solvent was removed by centrifugation, and the residue was washed sufficiently with pure water. Thus, a dispersion liquid of the magnetic particle including the silica layer, a first resin layer containing a fluorescent material, and a second resin layer was obtained. When the dispersion of the resultant magnetic particle was irradiated with a black light (manufactured by Nichia Corporation) having an excitation wavelength of 375 nm, red light emission was observed.

[Table 1-1]

| | | Solvent & Reducing agent | | $FeCl_3$ [M] | $FeCl_3 \cdot 6H_2O$ [M] | Externally added $H_2O$ [mL] | Total $H_2O$ [mL] | Sodium acetate [g] |
|---|---|---|---|---|---|---|---|---|
| | | Kind | Volume [mL] | | | | | |
| | Example 1 | Ethylene glycol | 30 | 0.15 | - | 0.40 | 0.40 | 2.7 |
| | Example 2 | Ethylene glycol | 30 | 0.15 | - | 0.40 | 0.40 | 2.7 |
| | Example 3 | Ethylene glycol | 30 | 0.15 | - | 0.40 | 0.40 | 2.7 |
| | Example 4 | Ethylene glycol | 30 | 0.15 | - | 0.40 | 0.40 | 2.7 |
| | Example 5 | Ethylene glycol | 30 | 0.15 | - | 0.40 | 0.40 | 2.7 |
| | Example 6 | Ethylene glycol | 30 | 0.15 | - | 0.40 | 0.40 | 2.7 |
| | Example 7 | Ethylene glycol | 30 | 0.15 | - | 0.40 | 0.40 | 2.7 |
| | Example 8 | Ethylene glycol | 30 | 0.03 | 0.12 | - | 0.39 | 2.7 |
| | Example 9 | Ethylene glycol | 30 | 0.03 | 0.12 | 0.40 | 0.79 | 2.7 |
| | Example 10 | Ethylene glycol | 30 | 0.03 | 0.12 | - | 0.39 | 2.7 |
| | Example 11 | Ethylene glycol | 30 | 0.12 | 0.03 | 0.40 | 0.50 | 2.7 |
| | Example 12 | Ethylene glycol | 30 | - | 0.15 | - | 0.49 | 2.7 |
| | Example 13 | Ethylene glycol | 30 | - | 0.15 | 0.40 | 0.89 | 2.7 |
| | Example 14 | Ethylene glycol | 30 | - | 0.15 | 0.40 | 0.89 | 2.7 |
| | Example 15 | Ethylene glycol | 30 | 0.15 | - | 0.40 | 0.40 | 2.7 |
| | Example 16 | Ethylene glycol | 30 | - | 0.15 | - | 0.49 | 2.7 |
| | Example 17 | Ethylene glycol | 30 | - | 0.15 | - | 0.49 | 2.7 |
| | Example 18 | Ethylene glycol | 30 | - | 0.15 | - | 0.49 | 2.7 |

(continued)

| | Solvent & Reducing agent | | FeCl$_3$ [M] | FeCl$_3$· 6H$_2$O [M] | Externally added H$_2$O [mL] | Total H$_2$O [mL] | Sodium acetate [g] |
|---|---|---|---|---|---|---|---|
| | Kind | Volume [mL] | | | | | |
| Example 19 | Ethylene glycol | 30 | - | 0.15 | - | 0.49 | 2.7 |
| Example 20 | Ethylene glycol | 30 | 1.5 | - | 0.40 | - | 2.7 |
| Example 21 | Ethylene glycol | 30 | 1.5 | - | 0.40 | - | 2.7 |
| Example 22 | Ethylene glycol | 30 | - | 0.15 | - | 0.49 | 2.7 |
| Example 23 | Ethylene glycol | 30 | - | 0.15 | - | 0.49 | 2.7 |
| Example 24 | Ethylene glycol | 30 | - | 0.15 | - | 0.49 | 2.7 |
| Example 25 | Ethylene glycol | 30 | - | 0.15 | - | 0.49 | 2.7 |
| Example 26 | Ethylene glycol | 30 | - | 0.15 | - | 0.49 | 2.7 |
| Example 27 | Ethylene glycol | 30 | - | 0.15 | - | 0.49 | 2.7 |
| Example 28 | Ethylene glycol | 30 | - | 0.15 | - | 0.49 | 2.7 |
| Example 29 | Ethylene glycol | 30 | - | 0.15 | - | 0.49 | 2.7 |
| Comparative Example 1 | Ethylene glycol | 30 | 0.15 | - | 0.40 | 0.40 | 2.7 |
| Comparative Example 2 | Ethylene glycol | 30 | 0.15 | - | - | 0.00 | 2.7 |
| Comparative Example 3 | Ethylene glycol | 30 | 0.15 | - | 8.50 | 8.50 | 2.7 |
| Comparative Example 4 | Ethylene glycol | 30 | 0.15 | - | 0.40 | 0.40 | 2.7 |
| Comparative Example 5 | 1,2-Propanediol | 30 | - | 0.15 | 0.40 | 0.89 | 2.7 |
| Comparative Example 6 | Ethylene glycol | 30 | 0.15 | - | 0.40 | 0.40 | 2.7 |

[Table 1-1] (continued)

| | Additive | | | Aging | | Heating | | |
|---|---|---|---|---|---|---|---|---|
| | Timing of addition | Kind | Concentration [M] | Temperature [°C] | Time [h] | Condition | Temperature [°C] | Time [h] |
| Example 1 | - | - | - | 25 | 72 | Static | 180 | 12 |
| Example 2 | - | - | - | 60 | 72 | Static | 180 | 12 |
| Example 3 | - | - | - | 100 | 72 | Static | 180 | 12 |
| Example 4 | - | - | - | 60 | 24 | Static | 180 | 12 |
| Example 5 | - | - | - | 60 | 120 | Static | 180 | 12 |
| Example 6 | - | - | - | 60 | 168 | Static | 180 | 12 |
| Example 7 | After aging | Trisodium citrate | 0.01 | 60 | 168 | Static | 180 | 12 |
| Example 8 | - | - | - | 60 | 168 | Static | 180 | 12 |
| Example 9 | - | - | - | 25 | 168 | Static | 180 | 12 |
| Example 10 | After aging | Trisodium citrate | 0.01 | 60 | 168 | Static | 180 | 12 |
| Example 11 | After aging | Sodium acrylate | 0.01 | 60 | 168 | Static | 180 | 12 |
| Example 12 | - | - | - | 25 | 72 | Static | 180 | 12 |
| Example 13 | - | - | - | 60 | 168 | Static | 180 | 12 |

(continued)

| | Additive | | | Aging | | Heating | | |
|---|---|---|---|---|---|---|---|---|
| | Timing of addition | Kind | Concentration [M] | Temperature [°C] | Time [h] | Condition | Temperature [°C] | Time [h] |
| Example 14 | Before aging | Sodium acrylate | 0.01 | 60 | 168 | Static | 180 | 12 |
| Example 15 | - | - | - | 60 | 168 | Stirring | 180 | 8 |
| Example 16 | Before aging | Disodium malate | 0.01 | 60 | 168 | Static | 180 | 12 |
| Example 17 | Before aging | Disodium malate | 0.08 | 60 | 168 | Static | 180 | 12 |
| Example 18 | Before aging | Sodium oleate | 0.01 | 60 | 168 | Static | 180 | 12 |
| Example 19 | Before aging | Sodium oleate | 0.08 | 60 | 168 | Static | 180 | 12 |
| Example 20 | Before aging | Sodium acrylate | 0.04 | 60 | 168 | Static | 180 | 12 |
| Example 21 | After aging | Sodium acrylate | 0.04 | 60 | 168 | Static | 180 | 12 |
| Example 22 | - | - | - | 60 | 4 | Static | 180 | 12 |
| Example 23 | - | - | - | 60 | 8 | Static | 180 | 12 |
| Example 24 | - | - | - | 60 | 12 | Static | 180 | 12 |
| Example 25 | - | - | - | 60 | 18 | Static | 180 | 12 |
| Example 26 | - | - | - | 60 | 24 | Static | 180 | 12 |
| Example 27 | - | - | - | 60 | 36 | Static | 180 | 12 |
| Example 28 | - | - | - | 60 | 48 | Static | 180 | 12 |
| Example 29 | - | - | - | 60 | 168 | Static | 180 | 12 |
| Comparative Example 1 | - | - | - | - | - | Static | 180 | 12 |
| Comparative Example 2 | - | - | - | 25 | 72 | Static | 180 | 12 |
| Comparative Example 3 | - | - | - | 60 | 168 | Static | 180 | 12 |
| Comparative Example 4 | - | - | - | 140 | 72 | Static | 180 | 12 |
| Comparative Example 5 | - | - | - | 60 | 168 | Static | 180 | 12 |
| Comparative Example 6 | - | - | - | - | - | Stirring | 180 | 8 |

[Table 1-2]

| | | Fe₃O₄ cluster | | | | |
|---|---|---|---|---|---|---|
| | Shape | Primary particle size [nm] | | Secondary particle size [nm] | | |
| | | SEM | XRD | SEM | DLS (D50) | DLS (D90) |
| Example 1 | Spherical | 13 | - | 832 | 535 | 742 |
| Example 2 | Spherical | 11 | - | 1,080 | 690 | 1,090 |
| Example 3 | Spherical | 8 | - | 1,240 | 678 | 1,340 |
| Example 4 | Spherical | 13 | 14 | 1,010 | - | - |
| Example 5 | Spherical | 12 | - | 1,330 | - | - |
| Example 6 | Spherical | 9 | 13 | 1,290 | 762 | 1,200 |
| Example 7 | Spherical | 10 | 11 | 934 | - | - |
| Example 8 | Spherical | 8 | 11 | 1,050 | - | - |
| Example 9 | Spherical | 16 | - | 913 | - | - |
| Example 10 | Spherical | 12 | - | 856 | - | - |
| Example 11 | Spherical | 11 | - | 622 | - | - |
| Example 12 | Spherical | 18 | - | 762 | 967 | 1,130 |
| Example 13 | Spherical | 19 | 15 | 2,160 | - | - |
| Example 14 | Spherical | 12 | 12 | 2,340 | 1,480 | 2,640 |
| Example 15 | Spherical | 5 | 9 | 625 | - | - |
| Example 16 | Spherical | - | 11 | - | 1,060 | 1,840 |
| Example 17 | Spherical | - | 7 | - | 608 | 856 |
| Example 18 | Spherical | - | 13 | - | 1,540 | 2,830 |
| Example 19 | Spherical | - | 11 | - | 510 | 690 |
| Example 20 | Spherical | - | 12 | - | 647 | 950 |
| Example 21 | Spherical | - | 11 | - | 512 | 784 |
| Example 22 | Spherical | - | 14 | - | 671 | 1,040 |
| Example 23 | Spherical | - | 14 | - | 813 | 1,330 |
| Example 24 | Spherical | - | 14 | - | 954 | 1,550 |
| Example 25 | Spherical | - | 13 | - | 781 | 1,260 |
| Example 26 | Spherical | - | 13 | - | 1,190 | 2,000 |
| Example 27 | Spherical | - | 12 | - | 1,200 | 2,100 |
| Example 28 | Spherical | - | 12 | - | 1,190 | 1,990 |
| Example 29 | Spherical | - | 14 | - | 1,110 | 1,790 |
| Comparative Example 1 | Spherical | 17 | 16 | 483 | 499 | 719 |
| Comparative Example 2 | Deformed and bound | 13 | 11 | 882 | - | - |
| Comparative Example 3 | Not aggregated | 38 | - | - | - | - |
| Comparative Example 4 | Irregular | 25 | - | - | - | - |
| Comparative Example 5 | Not aggregated | 28 | - | - | - | - |
| Comparative Example 6 | Spherical | 6 | 9 | 264 | - | - |

[Table 2-1]

| | Composition of Solution C | | | | Heating | | |
|---|---|---|---|---|---|---|---|
| | Solution A | | Solution B | | | | |
| | Volume fraction [%] | Volume [mL] | Volume fraction [%] | Volume [mL] | Condition | Temperature [°C] | Time [h] |
| Synthesis Example 1 | 100 | 30 | 0 | 0 | Static | 180 | 12 |
| Example 30 | 75 | 22.5 | 25 | 7.5 | Static | 180 | 12 |
| Example 31 | 50 | 15 | 50 | 15 | Static | 180 | 12 |
| Example 32 | 25 | 7.5 | 75 | 22.5 | Static | 180 | 12 |
| Synthesis Example 2 | 0 | 0 | 100 | 30 | Static | 180 | 12 |

[Table 2-2]

| | $Fe_3O_4$ cluster | | | | | |
|---|---|---|---|---|---|---|
| | Shape | Primary particle size [nm] | | Secondary particle size [nm] | | |
| | | SEM | XRD | SEM | DLS (D50) | DLS (D90) |
| Example 42 | Spherical | - | 17 | - | 543 | 933 |
| Example 43 | Spherical | - | 16 | - | 599 | 997 |
| Example 44 | Spherical | - | 16 | - | 697 | 1,070 |
| Example 45 | Spherical | - | 15 | - | 857 | 1,330 |
| Example 46 | Spherical | - | 14 | - | 1,030 | 1,710 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-145189 filed on September 7, 2023, Japanese Patent Application No. 2023-145346 filed on September 7, 2023, and Japanese Patent Application No. 2024-151501 filed on September 3, 2024, and the entire contents thereof are incorporated herein by reference.

[Reference Signs List]

11 primary particle for forming $Fe_3O_4$ cluster
12 $Fe_3O_4$ cluster in which a plurality of primary particles are aggregated
31 $Fe_3O_4$ cluster
32 inorganic layer
33 first resin layer
34 second resin layer
35 antibody
36 magnetic particle for assay

**Claims**

1.  A magnetic particle comprising a secondary particle in which a plurality of primary particles are aggregated,

    wherein an average particle size of the secondary particle is 500 nm or more and 5,000 nm or less, and
    wherein an average particle size of the primary particle is 2 nm or more and 20 nm or less.

2. The magnetic particle according to claim 1, wherein the average particle size of the secondary particle is 500 nm or more and 3,000 nm or less.

3. The magnetic particle according to claim 1 or 2, wherein the secondary particle contains $Fe_3O_4$.

4. The magnetic particle according to any one of claims 1 to 3, wherein a shape of the secondary particle is spherical.

5. The magnetic particle according to any one of claims 1 to 4, wherein magnetism of the secondary particle is superparamagnetism.

6. A magnetic particle for assay comprising:

    the magnetic particle of any one of claims 1 to 5; and
    a resin layer arranged outside the magnetic particle.

7. The magnetic particle for assay according to claim 6, further comprising a silica layer between the magnetic particle and the resin layer.

8. The magnetic particle for assay according to claim 6 or 7, wherein the resin layer includes at least a first resin layer and a second resin layer.

9. The magnetic particle for assay according to claim 8, wherein the first resin layer is a hydrophobic resin layer.

10. The magnetic particle for assay according to claim 8 or 9,

    wherein the second resin layer is arranged outside the first resin layer, and
    wherein the second resin layer is a polyglycidyl methacrylate layer.

11. The magnetic particle for assay according to any one of claims 6 to 10, further comprising a ligand bound to a surface of the resin layer.

12. The magnetic particle for assay according to claim 11, wherein the ligand is an antibody.

13. A method of producing a magnetic particle including a secondary particle in which a plurality of primary particles are aggregated, the method comprising:

    a step of obtaining an iron(III)-containing solution;
    a step of aging the iron(III)-containing solution;
    a step of obtaining a mixture in which the iron(III)-containing solution obtained in the step of aging and a precipitant are mixed; and
    a step of heating the mixture.

14. The method of producing a magnetic particle according to claim 13, wherein the iron(III)-containing solution obtained in the step of obtaining the iron(III)-containing solution contains ethylene glycol and iron(III) chloride anhydrous.

15. The method of producing a magnetic particle according to claim 13, wherein the iron(III)-containing solution obtained in the step of obtaining the iron(III)-containing solution contains ethylene glycol, iron(III) chloride anhydrous, and externally added water.

16. The method of producing a magnetic particle according to claim 13, wherein the iron(III)-containing solution obtained in the step of obtaining the iron(III)-containing solution contains ethylene glycol, iron(III) chloride anhydrous, and iron(III) chloride hexahydrate.

17. The method of producing a magnetic particle according to claim 13, wherein the iron(III)-containing solution obtained in the step of obtaining the iron(III)-containing solution contains ethylene glycol, iron(III) chloride hexahydrate, and externally added water.

18. The method of producing a magnetic particle according to any one of claims 13 to 17, wherein an aging time in the step of aging is 1 hour or more and 200 hours or less.

19. The method of producing a magnetic particle according to any one of claims 13 to 18, wherein an aging temperature in the step of aging is 10°C or more and 130°C or less.

20. The method of producing a magnetic particle according to any one of claims 14 to 17, wherein an amount of water per 30 mL of the ethylene glycol in the iron(III)-containing solution is 10.0 mL or less.

21. The method of producing a magnetic particle according to claim 20, wherein the amount of water per 30 mL of the ethylene glycol in the iron(III)-containing solution is 0.2 mL or more and 8.0 mL or less.

22. The method of producing a magnetic particle according to any one of claims 13 to 21, wherein the precipitant is an acetate.

# FIG. 1

11

12

FIG. 2

# FIG. 3

IRON(III)-CONTAINING
SOLUTION

AGING

PRECIPITANT

MIXED LIQUID

HEATING

$Fe_3O_4$ CLUSTER

# FIG. 4

WEAK AGING
IRON(III)-CONTAINING
SOLUTION

STRONG AGING
IRON(III)-CONTAINING
SOLUTION

PRECIPITANT

MIXED LIQUID

HEATING

$Fe_3O_4$ CLUSTER

# FIG. 5A

500 nm

# FIG. 5B

50 nm

# FIG. 6A

500 nm

# FIG. 6B

50 nm

## FIG. 7A

## FIG. 7B

# FIG. 8A

500 nm

# FIG. 8B

50 nm

## FIG. 9A

## FIG. 9B

# FIG. 10A

# FIG. 10B

FIG. 10C

FIG. 10D

## FIG. 10E

500 nm

## FIG. 10F

500 nm

# FIG. 10G

# FIG. 10H

FIG. 11A

FIG. 11B

## FIG. 12A

500 nm

## FIG. 12B

50 nm

# FIG. 13A

500 nm

# FIG. 13B

500 nm

FIG. 13C

FIG. 13D

# FIG. 13E

500 nm

# FIG. 14

## FIG. 15A

## FIG. 15B

## FIG. 15C

## FIG. 15D

# EP 4 773 156 A1

## INTERNATIONAL SEARCH REPORT

| International application No. |
|---|
| **PCT/JP2024/031799** |

### A. CLASSIFICATION OF SUBJECT MATTER

*H01F 1/36*(2006.01)i; *B82Y 5/00*(2011.01)i; *B82Y 40/00*(2011.01)i; *C01G 49/08*(2006.01)i; *G01N 33/531*(2006.01)i; *G01N 33/543*(2006.01)i; *G01N 33/545*(2006.01)i; *H01F 1/00*(2006.01)i; *H01F 1/11*(2006.01)i; *H01F 1/34*(2006.01)i; *H01F 1/44*(2006.01)i

FI: H01F1/36; C01G49/08 A; H01F1/11; B82Y5/00; B82Y40/00; H01F1/34 140; H01F1/44 150; H01F1/00 154; G01N33/531 B; G01N33/543 541A; G01N33/545 Z; H01F1/00 145

According to International Patent Classification (IPC) or to both national classification and IPC

### B. FIELDS SEARCHED

Minimum documentation searched (classification system followed by classification symbols)

B82Y5/00; B82Y40/00; C01G49/00; G01N33/531; G01N33/543; G01N33/545; H01F1/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 2023/056457 A1 (AGILENT TECHNOLOGIES, INC.) 06 April 2023 (2023-04-06) paragraphs [0032]-[0128], fig. 1A-13B | 1-5 |
| Y | paragraphs [0032]-[0128], fig. 1A-13B | 6-12 |
| A | paragraphs [0032]-[0128], fig. 1A-13B | 13-22 |
| Y | JP 2008-151761 A (CANON KABUSHIKI KAISHA) 03 July 2008 (2008-07-03) paragraphs [0015]-[0065], fig. 2-6 | 6-12 |
| Y | JP 2020-183946 A (CANON KABUSHIKI KAISHA) 12 November 2020 (2020-11-12) paragraphs [0012]-[0051] | 6-12 |
| Y | JP 2005-98974 A (SEKISUI CHEMICAL CO., LTD.) 14 April 2005 (2005-04-14) paragraphs [0007]-[0090] | 6-12 |

☑ Further documents are listed in the continuation of Box C.　　☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **17 October 2024** | **05 November 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

44

**INTERNATIONAL SEARCH REPORT**

| International application No. |
| --- |
| **PCT/JP2024/031799** |

C.    DOCUMENTS CONSIDERED TO BE RELEVANT

| Category* | Citation of document, with indication, where appropriate, of the relevant passages | Relevant to claim No. |
| --- | --- | --- |
| A | JP 2019-509975 A (AMOLIFESCIENCE CO., LTD.) 11 April 2019 (2019-04-11)<br>entire text, all drawings | 1-22 |
| A | JP 2014-532291 A (MAGFORCE AG) 04 December 2014 (2014-12-04)<br>entire text, all drawings | 13-22 |
| A | JP 2003-513156 A (FERROTEC CORP.) 08 April 2003 (2003-04-08)<br>entire text, all drawings | 13-22 |

Form PCT/ISA/210 (second sheet) (July 2022)

## INTERNATIONAL SEARCH REPORT
### Information on patent family members

International application No.

**PCT/JP2024/031799**

| Patent document cited in search report | | | Publication date (day/month/year) | Patent family member(s) | | | Publication date (day/month/year) |
|---|---|---|---|---|---|---|---|
| WO | 2023/056457 | A1 | 06 April 2023 | US | 2024/0025759 | A1 | |
| | | | | paragraphs [0029]-[0107], fig. 1A-10 | | | |
| | | | | WO | 2023/055385 | A1 | |
| | | | | CN | 117999619 | A | |
| | | | | KR | 10-2024-0069796 | A | |
| | | | | JP | 2024-537005 | A | |
| JP | 2008-151761 | A | 03 July 2008 | US | 2009/0000360 | A1 | |
| | | | | paragraphs [0036]-[0092], fig. 2-6 | | | |
| | | | | US | 2011/0136264 | A1 | |
| | | | | WO | 2007/114512 | A1 | |
| JP | 2020-183946 | A | 12 November 2020 | US | 2022/0034875 | A1 | |
| | | | | paragraphs [0012]-[0058] | | | |
| | | | | WO | 2020/218317 | A1 | |
| JP | 2005-98974 | A | 14 April 2005 | (Family: none) | | | |
| JP | 2019-509975 | A | 11 April 2019 | US | 2018/0254130 | A1 | |
| | | | | entire text, all drawings | | | |
| | | | | US | 2020/0265978 | A1 | |
| | | | | WO | 2018/034464 | A1 | |
| | | | | EP | 3389062 | A1 | |
| | | | | CN | 108496231 | A | |
| | | | | KR | 10-1729687 | B1 | |
| JP | 2014-532291 | A | 04 December 2014 | US | 2014/0154324 | A1 | |
| | | | | entire text, all drawings | | | |
| | | | | US | 2016/0367673 | A1 | |
| | | | | WO | 2013/020701 | A2 | |
| | | | | EP | 3038115 | A1 | |
| | | | | KR | 10-2014-0037290 | A | |
| | | | | CN | 104620334 | A | |
| JP | 2003-513156 | A | 08 April 2003 | US | 6277298 | B1 | |
| | | | | entire text, all drawings | | | |
| | | | | WO | 2001/031662 | A1 | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023145189 A
- JP 2023145346 A
- JP 2024151501 A

**Non-patent literature cited in the description**

- **S. XUAN** ; **Y. X. J. WANG** ; **J. C. YU** ; **K. C. F. LEUNG**. *Chemistry of Materials*, 2009, vol. 21, 5079-5087
- **J. LIU** ; **Z. SUN** ; **Y. DENG** ; **Y. ZOU** ; **C. LI** ; **X. GUO** ; **L. XIONG** ; **Y. GAO** ; **F. LI** ; **D. ZHAO**. *Angewandte Chemie*, 2009, vol. 48, 5875-5879